Europäisches Patentamt
European Patent Office
Office européen des brevets

(19)

(11) Veröffentlichungsnummer: **0 111 453**
**A1**

(12) # EUROPÄISCHE PATENTANMELDUNG

(21) Anmeldenummer: **83810572.4**

(22) Anmeldetag: **05.12.83**

(51) Int. Cl.[3]: **C 07 D 211/90**
C 07 D 491/04, C 07 D 409/04
C 07 D 405/04, C 07 D 401/04
A 61 K 31/445
//(C07D491/04, 307/00, 221/00)

(30) Priorität: **10.12.82 CH 7212/82**
**25.03.83 CH 1643/83**

(43) Veröffentlichungstag der Anmeldung:
**20.06.84 Patentblatt 84/25**

(84) Benannte Vertragsstaaten:
**AT BE CH DE FR GB IT LI LU NL SE**


(71) Anmelder: **CIBA-GEIGY AG**
**Postfach**
**CH-4002 Basel(CH)**

(72) Erfinder: **Kühnis, Hans, Dr.**
**Lange Gasse 26**
**CH-4052 Basel(CH)**


(54) **Amid-Verbindungen.**


(57) Die vorliegende Erfindung betrifft 2-Hydroxymethyl-3-carbamoyl-1,4-dihydro-pyridin-Verbindungen der Formel

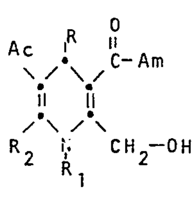

(I)

in welcher R einen carbocyclischen oder heterocyclischen Arylrest bedeutet, $R_1$ Wasserstoff oder unsubstituiertes oder substituiertes Niederalkyl bedeutet, $R_2$ Wasserstoff, Niederalkyl oder freies, veräthertes oder verestertes Hydroxy, funktionell abgewandeltes Carboxy oder freies oder substituiertes Amino enthaltendes Niederalkyl, funktionell abgewandeltes Carboxy oder freies oder substituiertes Amino bedeutet, wobei eine Aminogruppe $R_2$ mit einem Niederalkylrest $R_1$ verbunden sein kann, Ac den Acylrest einer Säure darstellt, und Am eine unsubstituierte oder monosubstituierte Aminogruppe bedeutet, mit der Massgabe, dass R von einem N-Oxido-pyridylrest verschieden ist, und mit der weiteren Massgabe, dass R von einem durch Halogen mit Atomnummer von höchstens 19 tetra- oder pentasubstituierten Phenylrest verschieden ist, wenn Ac für den Acylrest einer organischen Sulfonsäure steht, sowie Salze von Verbindungen der Formel I mit salzbildenden Eigenschaften, mit cardiovaskulären, insbesondere blutdrucksenkenden Eigenschaften, sowie Verfahren zur Herstellung dieser Verbindungen, ferner die Verwendung dieser Verbindungen und ihrer Salze und diese enthaltende pharmazeutische Präparate.


EP 0 111 453 A1

0111453



CIBA-GEIGY AG
Basel (Schweiz)

4-14365/14218/+

## Amid-Verbindungen

Die Erfindung betrifft neue Amid-Verbindungen und deren Salze, ferner Verfahren zu ihrer Herstellung, sowie diese enthaltende pharmazeutische Präparate und ihre Verwendung, oder als pharmakologisch aktive Verbindungen.
Die Erfindung betrifft 2-Hydroxymethyl-3-carbamoyl-1,4-dihydro-pyridin-Verbindungen der Formel

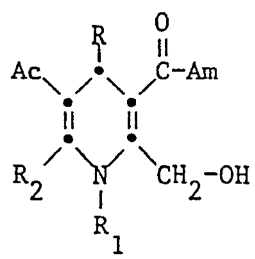

(I)

in welcher R einen carbocyclischen oder heterocyclischen Arylrest bedeutet, $R_1$ Wasserstoff oder unsubstituiertes oder substituiertes Niederalkyl bedeutet, $R_2$ Wasserstoff, Niederalkyl oder freies, veräthertes oder verestertes Hydroxy, funktionell abgewandeltes Carboxy oder freies oder substituiertes Amino enthaltendes Niederalkyl, funktionell abgewandeltes Carboxy oder freies oder substituiertes Amino bedeutet, wobei eine Aminogruppe $R_2$ mit einem Niederalkylrest $R_1$ verbunden sein kann, Ac den Acylrest einer Säure darstellt, und Am eine unsubstituierte oder monosubstituierte Aminogruppe bedeutet, mit der Massgabe, dass R von einem N-Oxido-pyridylrest verschieden ist, und mit der weiteren Massgabe, dass R von einem durch Halogen mit Atomnummer von höchstens 19 tetra- oder pentasubstituierten Phenylrest verschieden ist, wenn Ac für den Acylrest einer organischen Sulfonsäure steht, sowie Salze von Verbindungen der Formel I mit salzbildenden Eigenschaften.

Ein carbocyclischer oder heterocyclischer Arylrest ist in erster Linie ein entsprechender monocyclischer Rest, kann jedoch auch ein bi- oder polycyclischer, carbocyclischer oder heterocyclischer Rest mit aromatischen Eigenschaften sein.

Carbocyclische Reste dieser Art sind insbesondere Phenyl, ferner Naph-Naphthyl, z.B. 1- oder 2-Naphthyl.

Heterocyclische Arylreste sind vorzugsweise entsprechende monocyclische Reste, können jedoch auch entsprechende bi- oder polycyclische Reste sein, wobei letztere aus mehreren heterocyclischen Ringen, oder aus einem oder mehreren heterocyclischen Ringen mit einem oder mehreren ankondensierten carbocyclischen Ringen, insbesondere einem oder mehreren ankondensierten Benzoringen bestehen können. Die üblicherweise vorhandenen heterocyclischen Reste, die vorzugsweise aus fünf oder sechs Ringgliedern bestehen, können bis zu vier, gleiche oder verschiedene Hetero-, insbesondere Stickstoff-, Sauerstoff- und/oder Schwefelatome, vorzugsweise ein, zwei, drei oder vier Stickstoffatome, ein Sauerstoff- oder Schwefelatom, oder ein oder zwei Stickstoffatome zusammen mit einem Sauerstoff- oder Schwefelatom als Ringglieder enthalten. Sie sind mit dem 4-Ringkohlenstoffatom des 1,4-Dihydro-pyridinrings üblicherweise über ein Ringkohlenstoffatom gebunden.

Monocyclische fünfgliedrige Heteroarylreste sind z.B. entsprechende monoaza-, diaza-, triaza-, tetraza-, monooxa-, monothia-, oxaza-, oxadiaza-, thiaza- oder thiadiazacyclische Reste, wie Pyrryl-, Pyrazolyl-, Imidazolyl-, Triazolyl-, Tetrazolyl-, Furyl-, Thienyl-, Isoxazolyl-, Oxazolyl-, Oxadiazolyl-, Isothiazolyl-, Thiazolyl- oder Thiadiazolylreste, während monocyclische, sechsgliedrige Heteroarylreste z.B. entsprechende monoaza-, diaza- oder triazacyclische Reste, wie Pyridyl-, Pyridazinyl-, Pyrimidinyl-, Pyrazinyl- oder Triazinylreste sind. Bicyclische Heteroarylreste sind insbesondere monocyclische Heteroarylreste mit ankondensiertem Benzoring; der Heteroring kann fünf- oder

sechsgliedrig sein, wobei der fünfgliedrige Heteroarylrest z.B. einen monoaza-, diaza-, monooxa-, monothia-, oxaza- oder thiazacyclischen Rest, und der sechsgliedrige Heteroarylrest z.B. einen monoaza- oder einen diazacyclischen Heteroarylrest darstellt. Solche bicyclischen Reste sind z.B. Indolyl-, Isoindolyl-, Benzimidazolyl-, Benzofuranyl-, Benzothienyl-, Benzthiazolyl-, Chinolinyl- oder Isochinolinylreste.

Die carbocyclischen und heterocyclischen Arylreste R können unsubstituiert oder substituiert sein, wobei in erster Linie Ringkohlenstoffatome, aber auch Ringstickstoffatome substituiert sein können. Substituenten von Ringkohlenstoffatomen sind u.a. gegebenenfalls substituierte Kohlenwasserstoffreste, wie entsprechende aliphatische, cycloaliphatische, aromatische oder araliphatische Kohlenwasserstoffreste, wie Niederalkyl, Niederalkenyl, Niederalkinyl, Niederalkylen, Cycloalkyl, Cycloalkylniederalkyl, Phenyl oder Phenylniederalkyl, wobei Substituenten von solchen Kohlenwasserstoffresten, insbesondere von Niederalkyl, Phenyl oder Phenylniederalkyl, z.B. gegebenenfalls verätherte oder veresterte Hydroxygruppen, wie Hydroxy, Niederalkoxy, Niederalkenyloxy, Niederalkinyloxy, Niederalkylendioxy, Niederalkanoyloxy oder Halogen, und/oder gegebenenfalls funktionell abgewandeltes Carboxy, wie Carboxy, verestertes Carboxy, z.B. Niederalkoxycarbonyl, amidiertes Carboxy, wie Carbamoyl, N-Niederalkyl-carbamoyl oder N,N-Diniederalkyl-carbamoyl, oder Cyan sein können. Cyclische Substituenten, insbesondere Phenyl, können zudem auch Niederalkyl, das gegebenenfalls, z.B. wie angegeben, substituiert sein kann, als Substituenten enthalten. Weitere Substituenten von Arylresten R sind z.B. gegebenenfalls verätherte oder veresterte Hydroxygruppen, wie Hydroxy, Niederalkoxy, Halogenniederalkoxy, Niederalkenyloxy, Halogenniederalkenyloxy, Niederalkinyloxy, Niederalkylendioxy, Niederalkanoyloxy oder Halogen, oder gegebenenfalls veräthertes oder verestertes Hydroxy oder Mercapto enthaltendes Niederalkoxy, Niederalkenyloxy oder Niederalkinyloxy, wie Niederalkoxyniederalkoxy, Niederalkylthioniederalkoxy, Niederalkoxyniederalkenyloxy oder Niederalkylthioniederalkenyloxy, ferner Nitro, gegebenenfalls substituiertes Amino, wie Amino, N-Niederalkylamino, N,N-Diniederalkylamino, N-Niederalkyl-N-phenylniederalkylamino,

Niederalkylenamino, Oxaniederalkylenamino, Thianiederalkylenamino oder Azaniederalkylenamino, wobei der Aza-Stickstoff unsubstituiert oder z.B. durch gegebenenfalls, z.B. wie oben beschrieben, substituiertes Niederalkyl, Phenyl oder Phenylniederalkyl substituiert sein kann, oder Acylamino, z.B. Niederalkanoylamino, Azido, Acyl, wie Niederalkanoyl, oder gegebenenfalls funktionell abgewandeltes Carboxy, wie Carboxy, verestertes Carboxy, z.B. Niederalkoxycarbonyl oder amidiertes Carboxy, wie Carbamoyl, N-Niederalkyl-carbamoyl oder N,N-Diniederalkyl-carbamoyl, oder Cyan, gegebenenfalls funktionell abgewandeltes Sulfo, wie Sulfo oder Aminosulfonyl, und/oder veräthertes Mercapto, das gegebenenfalls oxidiert sein kann, wie Niederalkylthio, Niederalkylsulfinyl oder Niederalkylsulfonyl. Substituenten von Ringstickstoffatomen sind in erster Linie die obengenannten, gegebenenfalls substituierten Kohlenwasserstoffreste, wie Niederalkyl, ferner Hydroxy oder Oxido.

Heterocyclische Arylreste R können z.B. je nach Art der Substituenten in verschiedenartigen tautomeren Formen vorliegen.

Ein substituierter Niederalkylrest $R_1$ enthält als Substituenten in erster Linie unsubstituiertes oder insbesondere mono- oder disubstituiertes Amino, wie Diniederalkylamino, N-Niederalkyl-N-phenylniederalkyl-amino oder gegebenenfalls durch Sauerstoff, Schwefel oder unsubstituierten oder, z.B. durch Niederalkyl, substituierten Stickstoff unterbrochenes Niederalkylenamino, wobei ein solcher Substituent vorzugsweise durch mindestens 2 Kohlenstoffatome vom Ringstickstoffatom getrennt ist.

Veräthertes Hydroxy als Substituent eines Niederalkylrestes $R_2$ ist in erster Linie Niederalkoxy, während verestertes Hydroxy insbesondere Halogen oder Niederalkanoyloxy bedeutet. Funktionell abgewandeltes Carboxy als entsprechender Substituent einer Niederalkylgruppe $R_2$ ist insbesondere verestertes Carboxy, wie Niederalkoxycarbonyl, amidiertes Carboxy, wie Carbamoyl, N-Niederalkyl-carbamoyl oder N,N-Diniederalkylcarbamoyl, oder Cyan. Substituiertes Amino als Substituent von Niederalkyl $R_2$ ist monosubstituiertes Amino, wie Niederalkylamino, oder vorzugsweise disubstituiertes Amino, wie Diniederalkylamino, N-Niederalkyl-N-phenylniederalkyl-amino oder gegebenenfalls, z.B. durch Niederalkyl oder 2-Oxo-1-imidazolidinyl, substituiertes und/oder durch Sauerstoff, Schwefel oder gegebenenfalls, z.B. durch Niederalkyl oder Acyl, substituierten Stickstoff unterbrochenes Niederalkylenamino.

Funktionell abgewandeltes Carboxy und substituiertes Amino als Gruppe $R_2$ haben z.B. die oben gegebenen Bedeutungen und sind in erster Linie Niederalkoxycarbonyl oder Cyan, bzw. Niederalkylamino oder disubstituiertes Amino-niederalkylamino, wie Diniederalkylaminoniederalkylamino oder gegebenenfalls im Niederalkylenteil, z.B. durch Niederalkyl oder 2-Oxo-1-imidazolidinyl, substituiertes und/oder durch Sauerstoff, Schwefel oder gegebenenfalls, z.B. durch Niederalkyl oder Acyl, substituierten Stickstoff unterbrochenes Niederalkylenaminoniederalkylamino, ferner Diniederalkylamino, oder, gegebenenfalls im Niederalkylenteil, z.B. durch Niederalkyl, substituiertes und/oder durch Sauerstoff, Schwefel oder gegebenenfalls, z.B. durch Niederalkyl oder Acyl, substituierten Stickstoff unterbrochenes Niederalkylenamino.

Eine mit einem Niederalkylrest $R_1$ verbundene Aminogruppe $R_2$ kann mit diesem zusammen einen, über das Stickstoffatom mit dem Ringkohlenstoff verbundenen 1-Aza-niederalkylenrest, vorzugsweise mit 3 bis 5 Kettenatomen, bilden.

Ein Acylrest Ac kann den entsprechenden Rest einer Carbonsäure, insbesondere Niederalkanoyl, ferner unsubstituiertes oder substituiertes, z.B. Niederalkyl, Niederalkoxy und/oder Halogen enthaltendes Benzoyl, oder einer organischen Sulfosäure, insbesondere Niederalkylsulfonyl oder unsubstituiertes oder substituiertes, z.B. Niederalkyl, Niederalkoxy und/oder Halogen enthaltendes Phenylsulfonyl darstellen. Acylreste Ac stehen jedoch in erster Linie für Acylreste von Monoestern,

ferner von Monoamiden der Kohlensäure, wie unsubstituiertes oder substituiertes, z.B. freies oder veräthertes Hydroxy, wie Niederalkoxy, oder unsubstituiertes oder substituiertes Amino, wie z.B. oben beschriebenes Amino, und in erster Linie disubstituiertes Amino, wie Diniederalkylamino, N-Niederalkyl-N-phenylniederalkyl-amino, oder gegebenenfalls durch Sauerstoff, Schwefel oder unsubstituierten oder, z.B. durch Niederalkyl, substituierten Stickstoff unterbrochenes Niederalkylenamino, oder unsubstituiertes oder z.B. durch Niederalkyl, Niederalkoxy und/oder Halogen, substituiertes Phenyl, enthaltendes Niederalkoxycarbonyl, ferner N-unsubstituiertes oder N-mono- oder N,N-disubstituiertes Carbamoyl, wie N-Niederalkylcarbamoyl, N,N-Diniederalkylcarbamoyl, oder gegebenenfalls im Niederalkylenteil durch Sauerstoff, Schwefel oder unsubstituierten oder, z.B. durch Niederalkyl, substituierten Stickstoff unterbrochenes N,N-Niederalkylen-carbamoyl.

Eine Aminogruppe Am kann z.B. unsubstituiert oder N-unsubstituiert sein und bedeutet z.B. Amino, N-Niederalkyl-amino, z.B. Methylamino oder Aethylamino, N-(N'-mono-niederalkyl-amino)-niederalkylamino, z.B. (2-Methylamino-äthyl)-amino, oder -N-(N',N'-Diniederalkyl-amino)-niederalkyl-amino, z.B. (2-Dimethylamino-äthyl)-amino, ferner (2-Niederalkylenamino-äthyl)-amino, z.B. (2-Pyrrolidino-äthyl)-amino, (2-Piperidino-äthyl)-amino, [2-(4-Morpholino)-äthyl]-amino, oder [2-(4-Niederalkyl-piperazino)-äthyl]-amino, z.B. [2-(4-Methylpiperazino)-äthyl]-amino.

Die vor-, sowie nachstehend verwendeten Definitionen haben, sofern nicht besonders definiert, die folgenden Bedeutungen:

Der Ausdruck "nieder" bedeutet, dass entsprechend definierte Gruppen oder Verbindungen bis und mit 7, vorzugsweise bis und mit 4 Kohlenstoffatome enthalten.

Substituierte Reste können einen oder mehrere, gleiche oder verschiedene Substituenten enthalten; diese können irgendeine geeignete Stellung substituieren.

Naphthyl kann 1- oder 2-Naphthyl sein.

Pyrryl ist z.B. 2- oder 3-Pyrryl, Pyrazolyl z.B. 3- oder 4-Pyrazolyl, Imidazolyl z.B. 2- oder 4-Imidazolyl, Triazolyl z.B. 1,3,5-1H-Triazol-2-yl oder 1,3,4-Triazol-2-yl, und Tetrazolyl, z.B. 1,2,3,4-1H-Tetrazol-5-yl, während Furyl 2- oder 3-Furyl und Thienyl 2- oder 3-Thienyl bedeuten. Isoxazolyl ist z.B. 3-Isoxazolyl, Oxazolyl z.B. 2- oder 4-Oxazolyl, Oxadiazolyl z.B. 1,3,4-Oxadiazol-2-yl, Isothiazolyl z.B. 3-Isothiazolyl, Thiazolyl 2- oder 4-Thiazolyl, und Thiadiazolyl z.B. 1,3,4-Thiadiazol-2-yl.

Pyridyl ist 2-, 3- oder 4-Pyridyl, Pyridazinyl z.B. 3-Pyridazinyl, Pyrimidinyl ist 2-, 4- oder 5-Pyrimidinyl, Pyrazinyl ist 2-Pyrazinyl, und Triazinyl ist z.B. 1,3,5-Triazin-2-yl.

Indolyl ist z.B. 2-, 3- oder 5-Indolyl, Isoindolyl z.B. 1-Isoindolyl, Benzimidazolyl z.B. 2- oder 5-Benzimidazolyl, Benzofuranyl z.B. 2- oder 3-Benzofuranyl, Benzothienyl z.B. 3-Benzothienyl, Benzthiazolyl, z.B. 2-Benzthiazolyl, Chinolinyl z.B. 2- oder 4-Chinolinyl, und Isochinolinyl z.B. 1-Isochinolinyl.

Niederalkyl ist z.B. Methyl, Aethyl, n-Propyl, Isopropyl, n-Butyl, Isobutyl oder tert.-Butyl, ferner n-Pentyl, n-Hexyl oder n-Heptyl, während Niederalkenyl z.B. Allyl oder Methallyl, und Niederalkinyl z.B. Propargyl bedeutet.

Niederalkylen enthält z.B. drei bis fünf Kettenkohlenstoffatome und ist u.a. 1,3-Propylen oder 1,4-Butylen.

Cycloalkyl weist vorzugsweise 5 bis 7 Ringkohlenstoffatome auf und ist z.B. Cyclopentyl oder Cyclohexyl, während Cycloalkylniederalkyl z.B.

Cyclopropylmethyl, Cyclopentylmethyl oder Cyclohexylmethyl sein kann.

Phenylniederalkyl ist z.B. Benzyl oder 1- oder 2-Phenyläthyl.

Niederalkoxy steht in erster Linie für Methoxy, Aethoxy, n-Propyloxy, Isopropyloxy, n-Butyloxy, Isobutyloxy oder tert.-Butyloxy.

In einem Niederalkoxyniederalkoxy- oder einem Niederalkylthioniederalkoxyrest ist die terminale Niederalkoxygruppe vorzugsweise durch mehr als ein Kohlenstoffatom vom Verknüpfungskohlenstoffatom getrennt; solche Reste sind z.B. 2-Methoxy-äthoxy, 2-Aethoxy-äthoxy, 2-Methylthioäthoxy oder 2-Aethylthio-äthoxy.

Niederalkenyloxy ist z.B. Allyloxy oder Methallyloxy, und Halogenniederalkenyloxy, das ein oder mehrere Halogenatome enthalten kann, wobei letztere vorzugsweise eine Atomnummer bis und mit 35 aufweisen und besonders für Fluor und Chlor stehen, ist z.B. 1,2-Dichlor-vinyloxy.

Niederalkoxyniederalkenyloxy ist z.B. 2-Methoxy-vinyloxy, während Niederalkylthioniederalkenyloxy z.B. 2-Methylthio-vinyloxy ist.

In einem Halogen-niederalkoxyrest können ein oder mehrere Halogenatome, die vorzugsweise eine Atomnummer bis und mit 35 aufweisen und besonders für Fluor oder Chlor stehen, vorhanden sein; solche Reste sind z.B. Difluormethoxy oder 1,1,2-Trifluor-2-chlor-äthoxy.

Niederalkinyloxy ist z.B. Propargyloxy, während Niederalkylendioxy z.B. Methylendioxy oder Aethylendioxy bedeutet.

Niederalkanoyloxy ist z.B. Acetyloxy, Propionyloxy oder Pivaloyloxy.

Halogensubstituiertes Niederalkyl ist z.B. Trifluormethyl, 1,1,2-Trifluor-2-chlor-äthyl oder Chlormethyl.

Halogen hat vorzugsweise eine Atomnummer bis und mit 35 und ist insbesondere Fluor oder Chlor, ferner Brom, kann jedoch auch Iod sein.

Niederalkoxycarbonyl ist z.B. Methoxycarbonyl, Aethoxycarbonyl, n-Propyloxycarbonyl, Isopropyloxycarbonyl, n-Butyloxycarbonyl, Isobutyloxycarbonyl oder tert.-Butyloxycarbonyl.

N-Niederalkyl-carbamoyl ist u.B. N-Methyl-carbamoyl oder N-Aethylcarbamoyl, während N,N-Diniederalkyl-carbamoyl z.B. N,N-Dimethylcarbamoyl oder N,N-Diäthyl-carbamoyl ist.

N-Niederalkylamino ist z.B. N-Methylamino, N-Aethylamino, N-n-Propylamino oder N-Isopropylamino.

N,N-Diniederalkylamino ist z.B. N,N-Dimethylamino, N-Aethyl-N-methylamino oder N,N-Diäthylamino, während N-Niederalkyl-N-phenylniederalkylamino z.B. N-Benzyl-N-methylamino oder N-Methyl-N-(2-phenyläthyl)-amino darstellt.

N-(N'-(Mono -niederalkyl)-amino)-niederalkyl-amino ist z.B. N-[2-(Methylamino)-äthyl]-amino.

N-(N',N'-Di-(niederalkyl)-amino)-niederalkyl-amino ist z.B. N-[2-(Dimethylamino)-äthyl]-amino.

Niederalkylenamino enthält vorzugsweise 4 bis 6 Ringkohlenstoffatome und ist z.B. Pyrrolidino oder Piperidino, während Oxaniederalkylenamino z.B. Morpholino, wie 4-Morpholino, Thianiederalkylenamino, z.B. Thiomorpholino, wie 4-Thiomorpholino, und durch gegebenenfalls azasubstituiertes Azaniederalkylenamino z.B. Piperazino, 4-Methylpiperazino, 4-Phenyl-piperazino, 4-Benzyl-piperazino oder 4-(2-Phenyläthyl)-piperazino darstellen kann.

Niederalkanoylamino ist z.B. Acetylamino oder Propionylamino.

Niederalkanoyl ist z.B. Formyl, Acetyl, Propionyl oder Pivaloyl.

Niederalkylthio ist z.B. Methylthio, Aethylthio, n-Propylthio oder Isopropylthio, während Niederalkylsulfinyl z.B. Methylsulfinyl, und Niederalkylsulfonyl z.B. Methylsulfonyl oder Aethylsulfonyl bedeuten.

In einer Aminoniederalkylgruppe $R_1$ ist Amino vorzugsweise durch mindestens 2 Kohlenstoffatome vom Verknüpfungskohlenstoffatom getrennt; solche Reste sind in erster Linie 2-disubstituiertes Amino-niederalkyl, wie 2-Diniederalkylaminoäthyl, z.B. 2-Dimethylaminoäthyl oder 2-Diäthylaminoäthyl, 2-Niederalkylenaminoäthyl, z.B. 2-Pyrrolidino-äthyl oder 2-Piperidino-äthyl, 2-(4-Morpholino)-äthyl, oder 2-(4-Niederalkylpiperazino)-äthyl, z.B. 2-(4-Methylpiperazino)-äthyl.

Durch 2-Oxo-1-imidazolidinyl substituiertes Niederalkylenamino ist z.B. 4-(2-Oxo-1-imidazolidinyl)-piperidino.

Acyl als Substituent des, einen Niederalkylenaminorest unterbrechenden Stickstoffatoms ist in erster Linie der Acylrest einer Carbonsäure, wie Niederalkanoyl, z.B. Acetyl oder Propionyl, Benzoyl, Furoyl, z.B. 2-Furoyl, oder Thienoyl, z.B. 2-Thienoyl. Ein entsprechender Azaniederalkylenaminorest ist z.B. 4-Niederalkanoyl-piperazino, 4-Benzoylpiperazino, 4-(2-Furoyl)-piperazino oder 4-(2-Thenoyl)-piperazino.

Substituierte Niederalkylreste $R_2$ sind in erster Linie entsprechend substituierte Methylreste, z.B. Hydroxymethyl, Niederalkoxymethyl, Halogenmethyl, Niederalkoxycarbonylmethyl oder Cyanmethyl.

Diniederalkylaminoniederalkylamino ist z.B. 2-Dimethylaminoäthyl-amino, 2-Diäthylaminoäthyl-amino oder 3-Dimethylaminopropyl-amino, während entsprechend durch Sauerstoff, Schwefel oder gegebenenfalls substituierten Stickstoff unterbrochenes Niederalkylenamino-niederalkylamino z.B. 2-Pyrrolidinoäthyl-amino, 2-Piperidinoäthyl-amino, 2-(4-Morpholino)-äthyl-amino oder 2-(4-Methyl-piperazino)-äthyl-amino darstellt.

Ein 1-Azaniederalkylenrest, den eine Aminogruppe $R_2$ zusammen mit einem Niederalkylenrest $R_1$ bilden kann, ist in erster Linie 1-Aza-1,3-propylen, ferner 1-Aza-1,4-butylen, wobei in einem solchen Rest das Aza-Stickstoffatom mit dem 6-Ringkohlenstoffatom und das Verknüpfungskohlenstoffatom mit dem Ringstickstoffatom das 1,4-Dihydropyridinrings verbunden ist.

In einem substituierten Niederalkoxycarbonyl ist der Substituent üblicherweise durch mindestens 2, vorzugsweise durch 2 oder 3 Kohlenstoffatome vom Sauerstoff getrennt. Solche Reste sind z.B. Hydroxyniederalkoxycarbonyl, wie 2-Hydroxyäthoxycarbonyl oder 2,3-Dihydroxypropyloxycarbonyl, Niederalkoxy-niederalkoxycarbonyl, z.B. 2-Methoxyäthoxycarbonyl, Diniederalkylamino-niederalkoxycarbonyl, z.B. 2-Dimethylaminoäthoxycarbonyl, 2-Diäthylaminoäthoxycarbonyl oder 3-Dimethylaminopropyloxycarbonyl, Niederalkylenamino-niederalkoxycarbonyl, z.B. 2-Pyrrolidinoäthoxycarbonyl oder 2-Piperidinoäthoxycarbonyl, Morpholino-niederalkoxycarbonyl, z.B. 2-(4-Morpholino)-äthoxycarbonyl, oder 4-Niederalkyl-piperazino-niederalkoxycarbonyl, z.B. 2-(4-Methylpiperazino)-äthoxy carbonyl.

Phenylniederalkoxycarbonyl ist z.B. Benzyloxycarbonyl oder 2-Phenyläthoxy-carbonyl.

N,N-Niederalkylen-carbamoyl ist z.B. Pyrrolidinocarbonyl oder Piperidinocarbonyl, wobei entsprechende Reste, in welchen der Niederalkylenteil durch Sauerstoff, Schwefel oder unsubstituierten oder substituierten Stickstoff unterbrochen ist, z.B. 4-Morpholinocarbonyl, 4-Thiomorpholino-carbonyl, 1-Piperazinocarbonyl oder 4-Methyl-1-piperazino-carbonyl bedeutet.

Verbindungen der Formel I mit salzbildenden Gruppen, insbesondere entsprechenden basischen Gruppen, können in Form von Salzen, insbesondere von Säureadditionssalzen, in erster Linie entsprechenden pharmazeutisch

verwendbaren Säureadditionssalzen vorliegen. Solche Salze sind z.B. diejenigen mit Halogenwasserstoffsäuren, z.B. Chlorwasserstoffsäure oder Bromwasserstoffsäure, ferner Salpetersäure, Schwefelsäure oder Phosphorsäure, oder organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, ferner Aminosäuren, oder organischen Sulfonsäuren, wie gegebenenfalls Hydroxy enthaltende Niederalkansulfonsäuren, z.B. Methansulfonsäure, Aethansulfonsäure, 2-Hydroxyäthansulfonsäure oder Aethan-1,2-disulfonsäure, oder Arylsulfonsäuren, z.B. Benzolsulfonsäure, 4-Methyl-benzolsulfonsäure oder Naphthalin-2-sulfonsäure, oder mit anderen sauren organischen Substanzen, wie Ascorbinsäure.

Je nach Art der Substitution bzw. Substituenten können die Verbindungen der Formel I in Form von Gemischen von Racematen, oder in Form von Racematen oder optisch aktiven Antipoden vorliegen.

Die Verbindungen der Formel I und Salze von solchen Verbindungen mit salzbildenden Eigenschaften besitzen wertvolle pharmakologische Eigenschaften, vor allem im cardiovasculären Bereich. So wirken sie coronardilatierend, wie sich z.B. am isolierten, mit erhöhter $K^+$-Konzentration 40 mMol) perfundierten Meerschweinchenherzen nach Langendorff, in Konzentrationen ab etwa 0,01-0,1 µMol ($EC_{50}$-Werte: 0,02-0,15 µMol) nachweisen lässt. Sie zeigen auch antihypertensive Wirkungen, die sich beispielsweise an der renalhypertonischen Ratte in Anlehnung an die von Byrom und Wilson, J. Physiol. (London), Bd. 93, S. 301 (1938), Gerold et al., Helv. Physiol. Pharmacol. Acta, Bd. 24, S. 58 (1966), und Goldblatt et al., J. Exptl. Med., Bd. 59, S. 347 (1934) beschriebene Testanordnung in Dosen ab etwa 0,3-10 mg/kg p.o. nachweisen lassen. So bewirkt z.B. die während 4 Tagen erfolgende orale Verabreichung

einer Tagesdosis von 0,3 mg/kg 3-Aethylaminocarbonyl-2-hydroxymethyl-6-methyl-4-(2-nitro-phenyl)-1,4-dihydro-pyridin-5-carbonsäuremethylester an renalhypertonische Ratten am 4. Tag eine Blutdrucksenkung von 34 mm Hg, und die von 1 mg/kg auf die gleiche Weise und während des gleichen Zeitraums verabreichten 2-Hydroxymethyl-6-methyl-3-methylaminocarbonyl-4-(2-nitro-phenyl)-1,4-dihydropyridin-5-carbonsäure-äthylesters eine Blutdrucksenkung von 39 mm Hg. Die blutdrucksenkende Wirkung der neuen Verbindungen kann auch an der narkotisierten Katze bei intravenöser Verabreichung von Dosen ab etwa 0,01 mg/kg - 1 mg/kg gezeigt werden. So wird z.B. nach i.V. Applikation von 0,1 mg/kg 2-Hydroxymethyl-6-methyl-3-methylaminocarbonyl-4-(2-nitro-phenyl)-1,4-dihydro-pyridin-5-carbonsäure-äthylester unmittelbar nach der Injektion eine Blutdrucksenkung von 40 mm Hg beobachtet.

Die neuen Verbindungen zeichnen sich besonders auch dadurch aus, dass sie relativ zur coronardilatierenden Wirkung schwache negativ inotrope bzw. chronotrope Effekte aufweisen, welche sich beispielsweise durch eine Hemmung der Kontraktionskraft bzw. der Kontraktionsfrequenz des isolierten Meerschweinchenatriums nachweisen lassen. So bewirkt beispielsweise 2-Hydroxymethyl-6-methyl-3-methylaminocarbonyl-4-(2-nitrophenyl)-1,4-dihydropyridin-5-carbonsäure-äthylester in einer Konzentration von 3 µMol eine Abnahme der Kontraktionskraft um 25%, während eine beginnende negativ chronotrope Wirkung der gleichen Substanz bei einer Konzentration von 1 µMol beobachtet wird.

Die neuen Verbindungen zeichnen sich ferner durch verhältnismässig raschen Eintritt der pharmakologischen Wirkungen, chemische Beständigkeit und, im Vergleich zu den cardiovasculären Wirkungen, eine relativ niedrige Toxizität aus.

Verbindungen der Formel I und Salze von solchen Verbindungen mit salzbildenden Eigenschaften können daher z.B. als Antihypertensiva und Coronardilatatoren zur Behandlung von cardiovasculären Krankeitszuständen, wie Angina pectoris und ihre Folgen, Gefässspasmen und hohem Blutdruck Verwendung finden. Mit den neuen Verbindungen wird auch eine Reduzierung des peripheren Gefässwiderstandes erreicht, was bei einer Herzinsuffizienz zu einer Entlastung des Herzens und zu einer Erhöhung des Herzminutenvolumens führt, so dass die neuen Verbindungen ausserdem zur Behandlung der Herzinsuffizienz verwendet werden können.

Die neuen Verbindungen sind aber auch wertvolle Zwischenprodukte zur Herstellung anderer, insbesondere pharmazeutisch wirksamer Verbindungen.

Die Erfindung betrifft insbesondere Verbindungen der Formel I, in welchen R für einen mono- oder bicyclischen, carbocyclischen Arylrest oder für einen fünf- oder sechsgliedrigen, ein bis und mit vier Ringstickstoffatomen, ein Ringsauerstoff- oder Ringschwefelatom, oder ein oder zwei Ringstickstoffatome zusammen mit einem Ringsauerstoff oder einem Ringschwefelatom als Ringglieder enthaltenden, monocyclischen Heteroarylrest, der gegebenenfalls einen ankondensierten Benzoring enthält, steht, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzofuranyl, Benzothienyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl, das seinerseits wie angegeben substituiert sein kann, als Substituenten enthalten können, und/oder durch Hydroxy, Niederalkoxy, Halogenniederalkoxy, Niederalkenyloxy, Niederalkinyloxy, Niederalkylendioxy, Niederalkanoyloxy, Niederalkoxyniederalkoxy, Niederalkylthioniederalkoxy, Halogen, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Substituenten Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl, und/oder Cyan als Substituenten enthalten können, und/oder durch Niederalkanoylamino, Azido, Carboxy, Niederalkoxycarbonyl, Carbamoyl, N-Niederalkyl-carbamoyl, N,N-Diniederalkyl-carbamoyl, Cyan, Sulfo, Aminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl oder Cyan als Substituenten enthalten kann, oder durch Hydroxy oder Oxido substituiert sein können, $R_1$ Wasserstoff, Niederalkyl, Diniederalkylamino-niederalkyl, Niederalkylenaminoniederalkyl, Morpholinoniederalkyl, Thiomorpholino-niederalkyl, Piperazinoniederalkyl oder 4-Niederalkyl-piperazino-niederalkyl darstellt, $R_2$ für Wasserstoff, Niederalkyl, Hydroxyniederalkyl, Niederalkoxyniederalkyl, Halogenniederalkyl, Niederalkoxycarbonylniederalkyl, Carbamoylniederalkyl, N-Niederalkylcarbamoylniederalkyl, N,N-Diniederalkylcarbamoylniederalkyl, Cyanniederalkyl, Aminoniederalkyl, Niederalkylaminoniederalkyl, Diniederalkylaminoniederalkyl, Niederalkylenaminoniederalkyl, Morpholinoniederalkyl, Thiomorpholinoniederalkyl, Piperazinoniederalkyl, 4-Niederalkyl-piperazino-niederalkyl, Niederalkoxycarbonyl, Carbamoyl, N-Niederalkylcarbamoyl, N,N-Diniederalkylcarbamoyl, Cyan, Amino, Niederalkylamino, Diniederalkylamino-niederalkylamino, Niederalkylenamino-niederalkylamino, Morpholino-niederalkylamino, Diniederalkylamino, Niederalkylenamino, (2-Oxo-1-imidazolidinyl)-niederalkylenamino, Morpholino, Thiomorpholino, Piperazino, 4-Niederalkyl-piperazino, 4-Niederalkanoyl-piperazino, 4-Benzoyl-piperazino, 4-Furoylpiperazino oder 4-Thienyl-piperazino steht, wobei eine Aminogruppe $R_2$ mit einem Niederalkylrest $R_1$ verbunden sein und mit diesem zusammen einen 1-Aza-niederalkylenrest bilden kann, dessen Stickstoffatom mit dem 6-Ringkohlenstoffatom des 1,4-Dihydro-pyridinrings verbunden ist, der Rest Ac für Niederalkanoyl, unsubstituiertes oder durch Niederalkyl, Niederalkoxy und/oder Halogen substituiertes Benzoyl, Niederalkylsulfonyl, unsubstituiertes oder durch Niederalkyl, Niederalkoxy und/oder Halogen substituiertes Phenylsulfonyl, Niederalkoxycarbonyl, Hydroxyniederalkoxycarbonyl, Niederalkoxyniederalkoxycarbonyl, Aminoniederalkoxycarbonyl, Niederalkylaminoniederalkoxycarbonyl, Diniederalkylaminoniederalkoxycarbonyl, N-Niederalkyl-N-phenylniederalkyl-aminoniederalkoxycarbonyl, Niederalkylenaminoniederalkoxycarbonyl, Morpholinoniederalkoxycarbonyl, Thiomorpholinoniederalkoxycarbonyl, Piperazinoniederalkoxycarbonyl, 4-Niederalkyl-piperazino-niederalkoxycarbonyl, unsubstituiertes oder durch Niederalkyl, Niederalkoxy und/oder Halogen substituiertes Phenylniederalkoxycarbonyl, Carbamoyl, N-Niederalkyl-carbamoyl, N,N-Diniederalkylcarbamoyl, N,N-Niederalkylencarbamoyl, Morpholinocarbonyl, Thiomorpholinocarbonyl, Piperazinocarbonyl oder 4-Niederalkyl-piperazinocarbonyl steht, und die Gruppe Am Amino, N-Niederalkylamino, N-(N'-Mono-niederalkylamino)-niederalkyl-amino, N-(N',N'-Diniederalkyl-amino)-niederalkyl-amino, (2-Niederalkylenamino-äthyl)-amino oder [2-(4-Niederalkyl-piperazino)-äthyl]-amino bedeutet, mit der Massgabe, dass R von einem N-Oxidopyridylrest verschieden ist, und mit der weiteren Massgabe, dass R von einem durch Halogen mit Atomnummer von höchstens 19 tetra- oder pentasubstituierten Phenylrest verschieden ist, wenn Ac für den Acylrest einer organischen Sulfonsäure steht, oder Salze, insbesondere pharmazeutisch verwendbare Salze von solchen Verbindungen mit salzbildenden Gruppen, in erster Linie Säureadditionssalze, wie pharmazeutisch verwendbare Säureadditionssalze von solchen Verbindungen mit salzbildenden basischen Gruppen.

Die Erfindung betrifft in erster Linie Verbindungen der Formel I, worin R für Phenyl, das gegebenenfalls durch Niederalkyl, Phenyl und/oder

Phenylniederalkyl, wobei solche Reste ihrerseits Hydroxy, Niederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Niederalkylendioxy, Niederalkoxyniederalkoxy, Niederalkylthioniederalkoxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, Niederalkylthio, Niederalkylsulfinyl und/oder Niederalkylsulfonyl substituiert sein kann, oder für Pyrryl, Furyl, Thienyl oder Pyridyl, die gegebenenfalls wie ein Phenylrest R substituiert sein können, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten, $R_1$ Wasserstoff, Niederalkyl, Diniederalkylamino-niederalkyl, Niederalkylenaminoniederalkyl oder (4-Morpholino)-niederalkyl darstellt, wobei Diniederalkylamino, Niederalkylenamino bzw. 4-Morpholino durch mindestens zwei Kohlenstoffatome von Ringstickstoffatom getrennt sind, $R_2$ für Wasserstoff, Niederalkyl, Hydroxyniederalkyl, Halogenniederalkyl, Niederalkoxycarbonylniederalkyl, Cyanniederalkyl, Diniederalkylaminoniederalkyl, Niederalkoxycarbonyl, Cyan, Amino, (4-Morpholino)-niederalkylamino, Niederalkylenamino, (2-Oxo-1-imidazolidinyl)-niederalkylenamino oder 4-(2-Furoyl)-piperazino bedeutet, wobei eine Aminogruppe $R_2$ mit einem Niederalkylrest $R_1$ verbunden sein kann und mit diesem zusammen einen 1-Aza-niederalkylenrest bilden kann, dessen Azastickstoffatom mit dem 6-Ringkohlenstoffatom des 1,4-Dihydro-pyridinrings verbunden ist, der Rest Ac für Niederalkanoyl, Niederalkylsulfonyl, Niederalkoxycarbonyl, Hydroxyniederalkoxycarbonyl, Niederalkoxyniederalkoxycarbonyl, N,N-Diniederalkylaminoniederalkoxycarbonyl, N-Niederalkyl-N-phenylniederalkyl-amino-niederalkoxycarbonyl, N,N-Niederalkylenaminoniederalkoxycarbonyl, (4-Morpholino)-niederalkoxycarbonyl, Carbamoyl, N-Niederalkyl-carbamoyl, N,N-Diniederalkyl-carbamoyl, N,N-Niederalkylencarbamoyl, 4-Niederalkyl-piperazino-carbonyl oder 4-Morpholinocarbonyl steht, und der Rest Am für Amino, N-Niederalkyl-amino, N-(N'-Mononiederalkylamino)-niederalkylamino, N-(N',N'-Diniederalkylamino)-niederalkyl-amino oder (2-Niederalkylenamino-äthyl)-amino steht, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome enthalten, mit der Massgabe, dass R von einem durch Halogen mit Atomnummer von höchstens 19 tetra- oder pentasubstituierten Phenylrest verschieden ist, wenn Ac für Niederalkylsulfonyl steht, oder Salze, insbesondere pharmazeutisch verendbare Salze von solchen Verbindungen mit salzbildenden Gruppen, in erster Linie Säureadditionssalze, besonders pharmazeutisch verwendbare Säureadditionssalze von solchen Verbindungen mit salzbildenden basischen Aminogruppen.

Die Erfindung betrifft ganz besonders Verbindungen der Formel I, worin R für Phenyl oder Thienyl steht, das gegebenenfalls durch Niederalkyl, z.B. Methyl, Hydroxy, Niederalkoxy, z.B. Methoxy, Niederalkylendioxy, z.B. Methylendioxy, Niederalkoxyniederalkoxy, z.B. 2-Methoxyäthoxy, Niederalkylthioniederalkoxy, z.B. 2-Methylthioäthoxy, Halogen, z.B. Fluor oder Chlor, Trifluormethyl, Nitro, Niederalkanoylamino, z.B. Acetylamino, und/oder Cyano mono-, di- oder trisubstituiert sein kann, $R_1$ insbesondere Wasserstoff, ferner Niederalkyl, z.B. Methyl, 2-(Diniederalkylamino)-niederalkyl, besonders 2-Dimethylaminoäthyl, 2-(Niederalkylenamino)-niederalkyl, besonders 2-(Pyrrolidino)-äthyl oder 2-(Piperidino)-äthyl, oder 2-(4-Morpholino)-niederalkyl, besonders 2-(4-Morpholino)-äthyl bedeutet, $R_2$ Niederalkyl, besonders Methyl, Hydroxyniederalkyl, besonders Hydroxymethyl, Halogenniederalkyl, insbesondere Chlormethyl, 2-(Diniederalkylamino)-niederalkyl, insbesondere 2-(Diniederalkylamino)-äthyl, Niederalkoxycarbonyl, besonders Methoxycarbonyl oder Aethoxycarbonyl, Cyan, Amino, (4-Morpholino)-niederalkylamino, besonders 2-(4-Morpholino)-äthylamino, Niederalkylenamino, besonders Pyrrolidino, (2-Oxo-1-imidazolidinyl)-niederalkylenamino, besonders (4-(2-Oxo-1-imidazolidinyl)-piperidino, oder 4-(2-Furoyl)-piperazino bedeutet, wobei eine Aminogruppe $R_2$ mit einem Niederalkylrest $R_1$ verbunden und zusammen mit diesem einen 1-Azaniederalkylenrest, insbesondere 1-Aza-1,3-propylen, bilden kann, dessen Azastickstoffatom mit dem 6-Ringkohlenstoffatom

des 1,4-Dihydro-pyridinrings verbunden ist, der Rest Ac Niederalkanoyl, z.B. Acetyl, Niederalkylsulfonyl, z.B. Methylsulfonyl oder Aethylsulfonyl, Niederalkoxycarbonyl, besonders Methoxycarbonyl, Aethoxycarbonyl, n-Propyloxycarbonyl, Isopropyloxycarbonyl oder n-Butyloxycarbonyl, 2-Niederalkoxy-niederalkoxycarbonyl, insbesondere 2-Methoxyäthoxycarbonyl, Diniederalkylamino-niederalkoxycarbonyl, wie 2-Dimethylamino-äthoxycarbonyl, 2-Diäthylaminoäthoxy-carbonyloxycarbonyl oder 3-Dimethylaminopropyloxycarbonyl, N-Niederalkyl-N-phenylniederalkyl-amino-niederalkoxycarbonyl, z.B. 2-(N-Benzyl-N-methyl-amino)-äthoxycarbonyl, Carbamoyl, N-Niederalkyl-carbamoyl, z.B. N-Methylcarbamoyl, N,N-Diniederalkyl-carbamoyl, z.B. N,N-Dimethyl-carbamoyl, N,N-Niederalkylenamino-carbonyl, z.B. Pyrrolidino-carbonyl, 4-Niederalkyl-piperazino-carbonyl, z.B. 4-Methyl-piperazino-carbonyl, oder 4-Morpholino-carbonyl bedeutet, und der Rest Am für Amino, N-Niederalkylamino, z.B. Methylamino, N-(N'-Mono-niederalkylamino)-niederalkyl-amino, z.B. 2-(Methylamino)-äthylamino, oder N-(N',N'-Diniederalkyl-amino)-niederalkyl-amino, z.B. 2-(Dimethylamino-äthyl)-amino steht, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome enthalten, oder Salze, insbesondere pharmazeutisch verwendbare Salze von solchen Verbindungen mit salzbildenden Gruppen, in erster Linie Säureadditionssalze, wie pharmazeutisch verwendbare Säureadditionssalze von solchen Verbindungen mit salzbildenden basischen Gruppen.

Die Erfindung betrifft insbesondere Verbindungen der Formel I, worin R für unsubstituiertes oder vorzugsweise durch Niederalkyl, z.B. Methyl, Niederalkoxy, z.B. Methoxy, Niederalkoxyniederalkoxy, z.B. 2-Methoxy-äthoxy, Niederalkylthioniederalkoxy, z.B. 2-Methylthioäthoxy, Halogenniederalkoxy, z.B. Difluormethoxy, Halogen, z.B. Fluor oder Chlor, Trifluormethyl, Nitro und/oder Cyan mono- oder disubstituiertes Phenyl, 2- oder 3-Thienyl, in erster Linie für 2,3-Dimethyl-phenyl, 2,3- oder 3,4-Dichlorphenyl, 2- oder 3-Trifluormethyl-phenyl, 2- oder 3-Nitrophenyl, 2- oder 3-Cyan-phenyl, oder 2- oder 3-(2-Methoxyäthoxy)-phenyl oder 2- oder 3-(2-Methylthioäthoxy)-phenyl steht, $R_1$ Wasserstoff, ferner 2-(4-Morpholino)-äthyl bedeutet, $R_2$ in erster Linie Methyl, ferner Hydroxymethyl, Niederalkoxycarbonyl, z.B. Methoxycarbonyl, Cyan oder Amino bedeutet, der Rest Ac Niederalkanoyl, insbesondere Acetyl, oder Niederalkylsulfonyl, z.B. Aethylsulfonyl, in erster Linie jedoch Niederalkoxycarbonyl, z.B. Methoxycarbonyl, Aethoxycarbonyl, n-Propyloxycarbonyl, Isopropyloxycarbonyl oder Isobutyloxycarbonyl, ferner Carbamoyl, N-Niederalkylcarbamoyl, z.B. N-Methylcarbamoyl, oder N,N-Diniederalkyl-carbamoyl, z.B. N,N-Dimethylcarbamoyl, darstellt, und die Gruppe Am für Amino oder in erster Linie für N-Niederalkylamino, z.B. Methylamino, steht, wobei Niederalkyl, Niederalkoxy und Niederalkanoyl bis und mit 4 Kohlenstoffatome enthalten, oder Salze, insbesondere pharmazeutisch verwendbare Salze von solchen Verbindungen mit salzbildenden Gruppen, in erster Linie Säureadditionssalze, wie pharmazeutisch verwendbare Säureadditionssalze von solchen Verbindungen mit salzbildenden basischen Gruppen.

Die Erfindung betrifft insbesondere die in den Beispielen beschriebenen spezifischen Verbindungen.

Die Verbindungen der Formel I und Salze von solchen Verbindungen mit salzbildenden Eigenschaften können in an sich bekannter Weise hergestellt werden, indem man z.B.

a) eine Verbindung der Formel

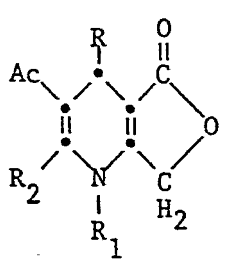

(II)

mit einer Verbindung der Formel H-Am (III) umsetzt, oder

b) in einer Verbindung der Formel

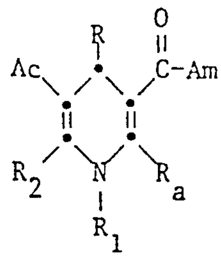

(IV)

in welcher $R_a$ einen in die Carbinolgruppe der Formel $-CH_2-OH$ überführbaren Rest bedeutet, den Rest $R_a$ in letztere überführt, oder

c) in einer Verbindung der Formel

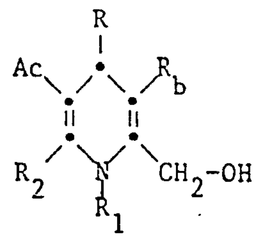

(V)

worin $R_b$ einen in die Carbamoylgruppe der Formel -C(=O)-Am überführbaren Rest darstellt, den Rest $R_b$ in letztere überführt, oder

d) in einer Verbindung der Formel

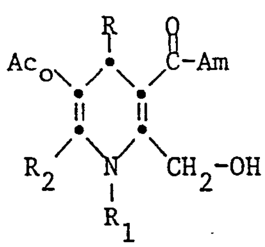

(VI)

worin $Ac_o$ einen in die Gruppe Ac überführbaren Rest darstellt, diesen in letztere überführt, oder

e) eine Verbindung der Formel

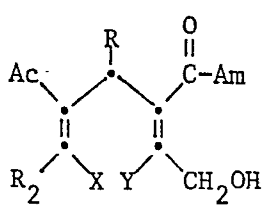

(VII),

worin einer der Reste X und Y für die Gruppe der Formel $-NH-R_1$ steht und der andere Hydroxy oder die Gruppe der Formel $-NH-R_1$ bedeutet, oder ein Tautomeres davon oder ein entsprechendes Tautomerengemisch

ringschliesst,
wobei in den obigen Ausgangsstoffen der Formeln II bis VII, die, sofern sie salzbildende Eigenschaften aufweisen, auch in Form ihrer Salze verwendet werden können, die Reste R, $R_1$, $R_2$, Ac und Am die unter der Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren in die einzelnen Isomeren auftrennt.

Die Verfahrensvariante a) wird üblicherweise in Gegenwart eines geeigneten inerten Lösungsmittels, wie eines Alkohols, z.B. Niederalkanols, wie Methanol oder Aethanol (wobei man bei Verwendung von Ausgangsstoffen der Formel II, die eine mit einem Alkohol, z.B. Niederalkanol, veresterte Carboxygruppe, z.B. als Gruppe Ac, aufweisen, den gleichen Alkohol als Lösungsmittel verwendet), und bei erniedrigter oder vorzugsweise bei erhöhter Temperatur, z.B. in einer Temperaturspanne von etwa 0°C bis etwa 150°C durchgeführt. Dabei arbeitet man bei Verwendung von leichtflüchtigen Verbindungen der Formel III vorzugsweise in einem geschlossenen Gefäss und, wenn notwendig, unter einer Inertgasatmosphäre.

Falls im Ausgangsmaterial der Formel II die Gruppen $R_2$ und Ac zusammen einen 2-Oxa-1-oxo-niederalkylenrest bilden, dessen Carbonylgruppe mit dem 5-Ringkohlenstoffatom des 1,4-Dihydro-pyridinrings verbunden ist, kann diese Gruppe unter den Reaktionsbedingungen durch Reaktion mit der Verbindung der Formel III ebenfalls aufgespalten werden; im entsprechenden Reaktionsprodukt der Formel I steht dann $R_2$ für eine Hydroxyniederalkylgruppe und Ac für eine amidierte Carboxygruppe. Ferner können gewisse, im Ausgangsmaterial vorhandene Substituenten, die sich leicht durch den Rest Am austauschen lassen, z.B. Halogen und in erster Linie Fluor in einem polyhalogenierten Phenylrest R, unter den Reaktionsbedingungen durch den Rest Am ausgetauscht werden.

Das Ausgangsmaterial der Formel II kann z.B. hergestellt werden, indem man eine Verbindung der Formel R-CHO (VIII) oder eines reaktionsfähigen Derivates davon, wie eines entsprechenden Acetals, z.B. eines Diniederalkyl-, wie Dimethyl- oder Diäthylacetals, mit einer Verbindung der Formel Ac-CH=C($R_2$)-NH-$R_1$ (IX) und einer Verbindung der Formel Hal-$CH_2$-C(=O)-$CH_2$-COOAlk (X), worin Hal für Halogen, wie Chlor oder Brom steht, und Alk Niederalkyl, z.B. Methyl oder Aethyl, darstellt, oder einem Tautomeren davon, umsetzt. Dabei erhält man unter den Reaktionsbedingungen direkt das Ausgangsmaterial der Formel II, das man ebenfalls durch Umsetzen einer Verbindung der Formel

R-CH=C(Ac)-CH($R_1$)-NH-$R_1$(XI) mit einer Verbindung der Formel

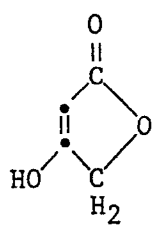

(XII)

oder einem Tautomeren davon bilden kann.

In einem Ausgangsmaterial der Formel IV bedeutet ein in die Carbinolgruppe überführbarer Rest $R_a$ z.B. ein mittels Reduktion oder Solvolyse in die Gruppe der Formel -$CH_2$-OH überführbarer Rest. Solche Reste $R_a$ sind u.a. Formyl, ferner verätherte oder veresterte Hydroxymethylgruppen. Ein Ausgangsmaterial der Formel IV mit einer Formylgruppe $R_a$ kann z.B. durch Behandeln mit einem geeigneten Reduktionsmittel, z.B. einem Hydridreduktionsmittel, wie einem Borhydrid-Reduktionsmittel, z.B. Natriumborhydrid, in die gewünschte Verbindung der Formel I umgewandelt werden. Verätherte Hydroxymethylgruppen $R_a$ sind z.B. Niederalkoxy, wie Methoxy, Aethoxy oder tert.-Butyloxy, ferner 2-Oxacycloalkoxy, z.B. 2-Tetrahydropyranyloxy; eine solche Gruppe $R_a$ kann z.B. durch Acidolyse, wie Behandeln mit einem geeigneten Aetherspaltungsmittel, z.B. einem Salz einer schwachen bis mittelstarken Base mit einer starken Mineralsäure, wie Pyridin-hydrochlorid, oder einem geeigneten Halogenid, wie einem Triniederalkyl-silylhalogenid, z.B. Trimethylsilyljodid, oder einem Bortrihalogenid, z.B.Bortribromid, 2-Oxacycloalkoxy

z.B. auch durch Behandeln mit einer Mineral-, wie Chlorwasserstoffsäure, gespalten und in die Hydroxymethylgruppe übergeführt werden. Veresterte Hydroxymethylgruppen $R_a$ sind z.B. Halogen-, wie Chlor- oder Brommethyl, oder Acyloxy-, wie Niederalkanoyloxy-, z.B. Acetyloxymethyl; Ausgangsstoffe der Formel IV mit solchen Gruppen $R_a$ können mittels Hydrolyse, üblicherweise in Gegenwart einer geeigneten Base, z.B. einer anorganischen Base, wie eines Alkalimetall-, z.B. Natrium- oder Kaliumhydroxids oder -carbonats, in Verbindungen der Formel I übergeführt werden.

Die obigen Reduktions- und Solvolysereaktionen werden in an sich bekannter Weise, wenn notwendig, in Anwesenheit eines Lösungs- oder Verdünnungsmittels, bei erniedrigter oder erhöhter Temperatur, z.B. in einem Temperaturbereich von etwa 0°C bis etwa 200°C, in einem geschlossenen Gefäss und/oder in einer Inertgasatmosphäre, durchgeführt.

Die Ausgangsstoffe der Formel IV können in an sich bekannter Weise hergestellt werden, z.B. indem man eine Verbindung der Formel XI mit einer Verbindung der Formel $Am-C(=O)-CH_2-C(=O)-R_a^o$ (XIII), worin $R_a^o$ den Rest $R_a$ oder eine in diese überführbare Gruppe darstellt, oder einem Tautomeren davon umsetzt, und in einer so erhältlichen Verbindung der Formel

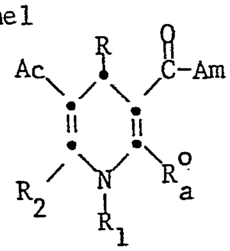

(IVa)

einen von $R_a$ verschiedenen Rest $R_a^o$ in letzteren überführt.

Eine von $R_a$ verschiedene Gruppe $R_a^o$ ist z.B. eine acetalisierte Formylgruppe, wie Diniederalkoxymethyl, z.B. Dimethoxymethyl oder Diäthoxymethyl.

Die Umsetzung einer Verbindung der Formel XI mit einer Verbindung der Formel XIII kann in an sich bekannter Weise, vorzugsweise bei erhöhter Temperatur, z.B. in einem Bereich von etwa 50° bis etwa 120° und in Gegenwart eines Lösungsmittels durchgeführt werden.

In einer Verbindung der Formel IVa, worin die Gruppe $R_a^o$ einen in die Gruppe $R_a$ überführbaren Rest darstellt, kann eine solche in an sich bekannter Weise umgewandelt werden, z.B. eine acetalisierte Formylgruppe durch Acidolyse, z.B. durch Behandeln mit Chlorwasserstoff in Gegenwart von Aceton.

In einer Verbindung der Formel V kann eine Gruppe $R_b$ z.B. eine freie Carboxygruppe oder eine, von einer amidierten Carboxygruppe verschiedene funktionell abgewandelte Carboxygruppe, z.B. eine Cyangruppe oder eine veresterte Carboxygruppe, wie Niederalkoxycarbonyl sein. Sie kann in an sich bekannter Weise, entweder direkt oder über eine Zwischenstufe, in die gewünschte amidierte Gruppe der Formel -C(=O)-Am umgewandelt werden.

Ein Ausgangsmaterial der Formel V, worin $R_b$ für Carboxy steht, kann z.B. mit einer Verbindung der Formel III, vorzugsweise in Gegenwart eines geeigneten Kondensationsmittels, wie einer Carbodiimid-Verbindung, z.B. N,N'-Dicyclohexyl-carbodiimid, umgesetzt werden. Oder ein solches Ausgangsmaterial kann zuerst durch Behandeln mit einem geeigneten Säurehalogenierungsmittel, wie Oxalylchlorid, in eine Verbindung der Formel V umgewandelt werden, worin $R_b$ für Halogen-, z.B. Chlorcarbonyl steht, und dann mit einer Verbindung der Formel III behandelt und so in die gewünschte Verbindung der Formel I übergeführt werden. Eine Verbindung der Formel V, worin $R_b$ eine Cyangruppe bedeutet, kann u.a. direkt zu einer Verbindung der Formel I, worin Am für Amino steht, z.B. durch Hydrolyse in Gegenwart eines basischen Mittels, wie eines Alkalimetall-, z.B. Natriumhydroxids, gegebenenfalls in Gegenwart von Wasserstoffperoxid, umgewandelt werden. Sie kann auch über eine entsprechende Iminoester- oder Iminothioester-Verbindung (wobei

in einer Verbindung der Formel V die Gruppe $R_b$ für einen Rest der Formel $-C(=NH)-X-R_c$ steht, worin X für Sauerstoff oder Schwefel und $R_c$ für einen organischen Rest, wie einen Kohlenwasserstoffrest, vorzugsweise Niederalkyl stehen), die man z.B. durch Umsetzen mit einem Alkohol oder einem Mercaptan in Gegenwart einer Säure, wie einer Mineralsäure, erhalten kann, oder über eine Amidin-Verbindung (wobei in einer Verbindung der Formel V die Gruppe $R_b$ für einen Rest der Formel $-C(=NH)-Am$ steht), die man durch Umsetzen einer Verbindung der Formel V, worin $R_b$ für Cyan oder für einen Rest der Formel $-C(=NH)-X-R_c$ steht, erhalten kann, gebildet werden. Die Umwandlung einer Iminoester-, Iminothioester- oder Amidin-Verbindung in eine Verbindung der Formel I kann durch Hydrolyse, z.B. unter sauren Bedingungen, wobei man u.a. Mineralsäuren verwenden kann, erfolgen. Dabei ergeben Iminoester- und Iminothioester-Ausgangsstoffe der Formel V Verbindungen der Formel I, worin Am eine unsubstituierte Aminogruppe darstellt, während Amidin-Ausgangsstoffe der Formel V zu Verbindungen der Formel I führen können, in denen Am gegebenenfalls substituiert ist.

Die obigen Reaktionen können in an sich bekannter Weise durchgeführt werden, wenn notwendig, in Gegenwart eines Lösungs- oder Verdünnungsmittels, bei erniedrigter oder erhöhter Temperatur, z.B. in einem Bereich von etwa 0°C bis etwa 120°C, in einem geschlossenen Gefäss und/oder unter einer Inertgasatmosphäre.

Die Ausgangsstoffe der Formel V können in an sich bekannter Weise hergestellt werden. So kann man z.B. eine Verbindung der Formel XI mit einer Verbindung der Formel $R_b^o-CH_2-C(=O)-R_a^o$ (XIV) oder einem Tautomeren davon umsetzen, wobei $R_b^o$ die Bedeutung von $R_b$ hat oder einen in die Gruppe $R_b$ überführbaren Rest darstellt. Letzterer kann z.B. eine unter milden Bedingungen in eine freie Carboxylgruppe überführbare veresterte Carboxygruppe sein, wie z.B. eine durch 2-Cyanäthyl oder Diphenylmethyl veresterte Carboxygruppe; erstere kann z.B. durch Behandeln mit einem etwa 1-n. wässrigen Alkalimetall-, z.B. Natriumhydroxid, und letzere durch Behandeln mit Ameisen- oder Trifluoressigsäure in

die freie Carboxylgruppe umgewandelt werden. In einer erhaltenen Verbindung kann, entweder vor oder nach der Umwandlung eines Restes $R_b^o$ in eine Gruppe $R_b$, die Gruppe $R_a^o$ (z.B. eine acetalisierte Formylgruppe), z.B. nach dem oben beschriebenen Verfahren, in eine Carbinolgruppe umgewandelt werden.

Ausgangsstoffe der Formel VI können entsprechend dem in ihnen enthaltenen Rest $Ac_o$ beispielsweise Carbonsäuren ($Ac_o$ ist Carboxyl), Carbonsäureanhydride, insbesondere gemischte Anhydride, wie Säurehalogenide, z.B. -chloride oder -bromide ($Ac_o$ ist Halogencarbonyl, z.B. Chlor- oder Bromcarbonyl), weiter aktivierte Ester, z.B. Cyanmethyl- oder Pentachlorphenylester ($Ac_o$ ist Cyanmethoxycarbonyl oder Pentachlorphenyloxycarbonyl), sein. Solche Ausgangsstoffe können, gegebenenfalls in Gegenwart von Kondensationsmitteln, durch Behandeln mit einem Alkohol, wie einem unsubstituierten oder substituierten Niederalkanol, oder einem reaktionsfähigen Derivat davon, z.B. einem entsprechenden Alkoholat, wie Alkalimetallalkoholat, freie Carbonsäuren auch durch Umsetzen mit geeigneten Diazo-Verbindungen, wie unsubstituierten oder substituierten Diazoniederalkanen, in Verbindungen der Formel I umgewandelt werden, in denen Ac den Acylrest eines Monoesters der Kohlensäure darstellt. Solche Verbindungen können ebenfalls erhalten werden, wenn als Ausgangsstoffe der Formel VI Salze, insbesondere Alkalimetall- oder Erdalkalimetallsalze, der freien Carbonsäure verwendet und diese mit reaktionsfähigen Estern von Alkoholen, wie unsubstituierten oder substituierten Niederalkanolen, wie entsprechenden Halogeniden, z.B. Chloriden, Bromiden oder Iodiden, oder organischen Sulfonsäureestern, z.B. Niederalkansulfonsäure- oder Arensulfonsäureestern, wie Methansulfonsäure- bzw. p-Toluolsulfonsäureestern, behandelt werden, oder wenn man entsprechende hydrolysierbare Iminoester, wie entsprechende Imino-niederalkylester, zu den Estern hydrolysiert.

Die Umsetzung von freien Carbonsäuren mit Alkoholen, wie unsubstituierten oder substituierten Niederalkanolen, erfolgt vorteilhaft in Gegenwart eines sauren, wasserabspaltenden Katalysators, wie einer Protonensäure, z.B. Chlorwasserstoff-, Bromwasserstoff-, Schwefel-, Phosphor- oder Borsäure, Benzolsulfon- oder Toluolsulfonsäure, oder einer Lewissäure, z.B. Bortrifluorid-Aetherat, in einem Ueberschuss des eingesetzten Alkohols und/oder in einem inerten Lösungsmittel, erforderlichenfalls unter destillativer, z.B. azeotroper, Entfernung des bei der Reaktion freigesetzten Wassers. Weiter kann man die Umsetzungen auch in Gegenwart von wasserbindenden Kondensationsmitteln, wie geeignet substituierten Carbodiimiden, z.B. N,N'-Diäthyl-, N,N'-Dicyclohexyl- oder N-Aethyl-N'-(3-dimethylaminopropyl)-carbodiimid, gegebenenfalls in inerten organischen Lösungsmitteln, durchführen. Gemischte Anhydride, insbesondere Säurehalogenide, werden beispielsweise in Gegenwart säurebindender Mittel, z.B. organischer, insbesondere tertiärer Stickstoffbasen, wie Triäthylamin, Aethyldiisopropylamin oder Pyridin, oder auch anorganischer Basen, z.B. von Alkalimetall- oder Erdalkalimetallhydroxiden oder -carbonaten, wie Natrium-, Kalium- oder Calciumhydroxid bzw. -carbonat, mit Alkohlen oder mit Alkoholaten, z.B. Alkalimetallniederalkoxiden, umgesetzt.

Die Umsetzungen von reaktionsfähigen Estern, z.B. Cyanmethyl- oder Pentachlorphenylestern, mit Alkoholen werden beispielsweise in einem gegenüber den Reaktionsteilnehmern inerten Lösungsmittel und im Temperaturbereich von etwa 0° bis etwa 120°C, vorzugsweise bei Raumtemperatur bis etwa 60°C, durchgeführt.

Die Hydrolyse von Iminoester-, insbesondere Iminoniederalkylester-Ausgangsstoffen, erfolgt beispielsweise mittels wasserhaltiger Mineralsäuren, wie Salzsäure oder Schwefelsäure, wobei man z.B. die bei der Addition von Chlorwasserstoff an Nitrile und Umsetzung mit wasserfreien Alkoholen, insbesondere unsubstituierten oder substituierten Niederalkanolen, erhaltenen Iminoestersalze, z.B. -hydrochloride, nach Zusatz von Wasser direkt zu den entsprechenden Estern hydrolysieren kann. Man kann z.B. auch aus einem Gemisch des Nitril-Ausgangsmaterials, eines Alkohols und Schwefelsäure mit geeignetem Wassergehalt, ohne Isolierung des in situ entstandenen Iminoesters, die gewünschte Esterverbindung der Formel I erhalten.

Verbindungen der Formel I, in denen mindestens der Rest Ac den Acylrest eines Kohlensäuremonoamids darstellt, kann man aus Verbindungen der Formel VI erhalten, in denen $Ac_o$ für eine Carboxylgruppe, eine Säureanhydridgruppe, wie Halogencarbonyl, z.B. Chlorcarbonyl, oder eine aktivierte Estergruppe, wie Cyanmethoxycarbonyl oder Pentachlorphenyloxycarbonyl, steht, indem man solche Ausgangsstoffe, gegebenenfalls in Gegenwart eines geeigneten Kondensationsmittels, mit Ammoniak oder einem Ammoniak abgebenden Mittel oder einem N-mono- oder N,N-disubstituierten Amin umsetzt.

Diese Umwandlungen von Carboxy- und geeignet funktionell abgewandelten reaktionsfähigen Carboxygruppen in gegebenenfalls N-mono- oder N,N-disubstituierte Carbamoylgruppen können in an sich bekannter Weise, z.B. nach den für die Bildung der Estergruppen beschriebenen Verfahren, durchgeführt werden.

Verbindungen der Formel I, in welchen die Gruppe Ac für Carbamoyl steht, kann man, ausgehend von Verbindungen der Formel VI, in welchen der Rest $Ac_o$ Cyan darstellt, ebenfalls erhalten. Solche Ausgangsstoffe können hydrolytisch, vorzugsweise unter sauren oder basischen Bedingungen, z.B. in Gegenwart eines Alkalimetall-, wie Natriumhydroxids, und, wenn erwünscht, von Wasserstoffperoxid, in einem wässrig-alkoholischen Lösungsmittel, wie wässrigem Aethanol, in die gewünschten Verbindungen der Formel I mit einer Carbamoylgruppe Ac übergeführt werden.

Die obigen Reaktionen können unter an sich bekannten Reaktionsbedingungen durchgeführt werden, in Ab- oder üblicherweise Anwesenheit von Lösungs- oder Verdünnungsmitteln, je nach Art der Reaktion und/oder Reaktionsteilnehmer bei erniedrigter oder erhöhter Temperatur, z.B. im Temperaturbereich von etwa -10°C bis etwa 150°C, unter atmosphärischem Druck oder in einem geschlossenen Gefäss, gegebenenfalls unter Druck, und/oder in einer inerten Atmosphäre, z.B. unter einer Stickstoffatmosphäre.

Ausgangsstoffe der Formel VI mit freier Carboxylgruppe $Ac_o$ können z.B. erhalten werden, indem man den entsprechenden 2-Cyanoäthylester herstellt und diesen, unter milden Bedingungen, z.B. mittels wässrigem oder wässrig-niederalkanolischem 1-n. Natriumhydroxid bei Raumtemperatur, zur freien Carbonsäure spaltet. Den 2-Cyanäthylester selber kann man z.B. durch Umsetzen einer Verbindung der Formel VIII mit einer Verbindung der Formel IX, worin Ac für eine 2-Cyanäthyloxycarbonylgruppe steht, und einer Verbindung der Formel X, oder einer Verbindung der Formel XI, worin Ac für eine 2-Cyanäthyloxycarbonylgruppe steht, mit einer Verbindung der Formel XII oder einem Tautomeren davon, und nachträglichem Spalten der im so erhältlichen Zwischenprodukt vorhandenen 2-Oxa-1-oxo-1,3-propylengruppe durch Behandeln mit einer Verbindung der Formel III. Die im Ausgangsmaterial der Formel VI vorhandene freie Carboxylgruppe kann, falls notwendig, in an sich bekannter Weise in die gewünschte reaktionsfähige, funktionell abgewandelte Form überführt werden.

Die als Ausgangsstoffe für die Verfahrensvariante d) ebenfalls in Betracht kommenden Nitrilverbindungen der Formel VI können z.B. analog zu den obgenannten 2-Cyanäthylester-Verbindungen hergestellt werden, indem man Zwischenprodukte verwendet, die anstelle des Restes Ac eine Cyangruppe enthalten.

Das Ringschlussverfahren gemäss Variante e) kann in an sich bekannter Weise durchgeführt werden, falls notwendig, in Gegenwart eines Kondensationsmittels, insbesondere eines basischen Kondensationsmittels, wie einer organischen Base, wie Piperidin oder Aethyl-diisopropyl-amin, oder eines Metallalkoholats, wie Alkalimetall-nieder-alkoxids, und/oder eines geeigneten Dehydratisierungs- oder wasseraufnehmenden Mittels, ferner üblicherweise in Gegenwart eines inerten organischen Lösungsmittels und bei Reaktionstemperaturen im Bereich von etwa Raumtemperatur bis etwa 150°C, insbesondere bei Siedetemperatur des Lösungsmittels. Gegebenenfalls erfolgt die Umsetzung in einer Inertgas-, z.B. Stickstoffatmosphäre, und/oder im geschlossenen Gefäss unter erhöhten Druck.

Die Ausgangsstoffe der Formel VII können in an sich bekannter Weise hergestellt werden, z.B. indem man in einer Verbindung der Formel

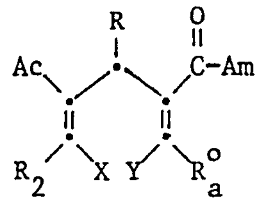

(XV),

worin $R_a^o$ die oben gegebene Bedeutung hat und z.B. insbesondere für eine acetalisierte Formylgruppe, wie Dimethoxymethyl oder Diäthoxymethyl, steht, den Rest $R_a^o$, z.B. mittels saurer hydrolytischer Spaltung der Acetalgruppe und nachfolgender Reduktion der Formylgruppe, wie nach dem oben beschriebenen Verfahren, in die gewünschte Carbinolgruppe überführt.

Verbindungen der Formel XV können in an sich bekannter Weise hergestellt werden, z.B. durch Umsetzen einer Verbindung der Formel VIII mit einer Verbindung der Formel IX und einer Verbindung der Formel XIII, wobei die Verbindungen der Formeln IX und/oder XIII in Form von Tautomeren oder Tautomerengemischen vorliegen können, und die Reaktionsbedingen so gewählt werden müssen, dass beim Ringschluss zu einer 1,4-Dihydro-pyridin-Verbindung mit einer acetalisierten Formylgruppe $R_a^o$ in 2-Stellung gebildet wird.

Erfindungsgemäss erhältliche Verbindungen der Formel I können in an sich bekannter Weise in andere Verbindungen der Formel I umgewandelt werden.

So kann man z.B. in Verbindungen der Formel I, worin $R_1$ für Wasserstoff steht, durch Behandeln mit einem reaktionsfähigen Ester eines Alkohols der Formel $R_1$-OH (XIV) einen organischen Rest $R_1$ einführen und so zu Verbindungen der Formel I gelangen, worin $R_1$ von Wasserstoff verschieden ist.

Reaktionsfähige Ester von Verbindungen der Formel XIV, z.B. von unsubstituierten oder substituierten Niederalkanolen, sind solche mit

starken, anorganischen oder organischen Säuren; dabei kommen beispielsweise die entsprechenden Halogenide, insbesondere Chloride, Bromide oder Jodide, ferner Sulfate, weiter Niederalkansulfonsäure- oder Arensulfonsäureester, z.B. Methansulfonsäure-, Benzolsulfonsäure- oder p-Toluolsulfonsäureester, in Betracht. Die Umsetzung wird, falls notwendig, unter Kühlen oder Erwärmen, z.B. in einem Temperaturbereich von etwa 0°C bis etwa 100°C, in Gegenwart eines geeigneten basischen Kondensationsmittels, z.B. eines Alkalimetalls, Alkalimetallamids oder -hydrids, oder eines Alkalimetallniederalkoxids, wie Natrium- oder Kalium-methoxid, -äthoxid oder tert.-butoxid, in An- oder Abwesenheit eines Lösungs- oder Verdünnungsmittels, bei erniedrigter oder erhöhter Temperatur, z.B. in einem Temperaturbereich von etwa 0°C bis etwa 100°C, und/oder unter atmosphärischem Druck oder in einem geschlossenen Gefäss durchgeführt.

Vorzugsweise verwendet man in solchen N-Substituierungsreaktionen in erster Linie Verbindungen der Formel I, die keine anderen Aminogruppen als Substituenten aufweisen, da diese unter Umständen ebenfalls mit dem reaktionsfähigen Ester eines Alkohols der Formel XIV in Reaktion treten können.

Ferner können in verfahrensgemäss erhältlichen Verbindungen der Formel I vorhandene Substituenten in andere Substituenten umgewandelt werden.

So kann man z.B. veresterte Carboxygruppen, wie entsprechende Gruppen Ac und/oder $R_2$ oder entsprechende Substituenten einer Gruppe $R_2$, durch Umesterung in andere Ester überführen. Dabei verwendet man vorzugsweise entsprechende Alkoholverbindungen, die einen Siedepunkt aufweisen, der deutlich über demjenigen des Alkohols der veresterten Gruppe in der umzuwandelnden Verbindung der Formel I liegt, und führt die Reaktion z.B. in einem Ueberschuss der Hydroxyverbindung und/oder einem inerten organischen, vorzugsweise ebenfalls deutlich über dem Alkohol der veresterten Gruppe siedenden Lösungsmittel, vorzugsweise

in Gegenwart eines Katalysators, z.B. eines Alkalimetall-niederalkoxids, wie Natrium- oder Kaliummethoxid oder -äthoxid, in der Wärme und üblicherweise unter Abdestillieren des freigesetzten Alkohols durch.

Verbindungen der Formel I mit veresterten Carboxygruppen, wie Niederalkoxycarbonylgruppen, insbesondere entsprechenden Gruppen Ac, können in solche mit entsprechenden Carboxamidgruppen umgewandelt werden, z.B. durch Behandeln mit Ammoniak, ferner mono- oder disubstituierten Aminen, wenn notwendig, unter erhöhter Temperatur und/oder in einem geschlossenen Gefäss.

Ferner kann man Verbindungen der Formel I, worin der Rest $R_2$ für eine 2-Aminoniederalkylgruppe, insbesondere eine 2-N,N-disubstituierte Amino-äthylgruppe steht, auch dadurch erhalten, dass man eine Verbindung der Formel I, worin der Rest $R_2$ für Niederalkyl, insbesondere Methyl steht, mit Formaldehyd oder einem solchen abgebenden Mittel, wie Paraformaldehyd, und einem Amin, insbesondere einem N,N-disubstituierten Amin, nach dem Mannich-Verfahren umsetzt.

Geeignete Substituenten eines aromatischen Restes, z.B. Halogen-, insbesondere Fluoratome, können gegen andere Substituenten, z.B. eine gegebenenfalls substituierte Aminogruppe (z.B. durch Behandeln mit einer Verbindung der Formel III) ausgetauscht werden.

Je nach Reaktionsbedingungen können die Verbindungen der Formel I in freier Form oder in Form von Salzen erhalten werden.

Erhaltene Säureadditionssalze können in an sich bekannter Weise in die freien Verbindungen, z.B. durch Behandeln mit einer Base, wie einem Alkalimetallhydroxid, oder in andere Salze, z.B. durch Behandeln mit geeigneten Säuren oder Derivaten davon, umgewandelt werden. Erhaltene freie Verbindungen mit salzbildenden Eigenschaften, z.B. mit

entsprechenden basischen Gruppen, können z.B. durch Behandeln mit Säuren oder entsprechenden Anionenaustauschern in ihre Salze umgewandelt werden.

Infolge der engen Beziehung zwischen den Verbindungen der Formel I in freier Form und in Form von Salzen, sind im Vorausgegangenen und nachfolgend unter den freien Verbindungen oder ihren Salzen sinn- und zweckmässig gegebenenfalls auch die entsprechenden Salze bzw. freien Verbindungen zu verstehen.

Die Verbindungen, einschliesslich ihre Salze, können auch in Form ihrer Hydrate erhalten werden, oder ihre Kristalle können z.B. das zur Kristallisation verwendete Lösungsmittel einschliessen.

Die Verbindungen der Formel I können, je nach Verfahrensreaktion und/oder Art der Ausgangsstoffe in Form von Racematen, Racematgemischen oder optischen Antipoden erhalten werden.

Erhaltene Racematgemische können auf Grund der physikalisch-chemischen Unterschiede der Racemate in bekannter Weise in die reinen Racemate bzw. Diastereomeren aufgetrennt werden, beispielsweise durch Chromatographie und/oder fraktionierte Kristallisation.

Racemate lassen sich nach an sich bekannten Methoden in die optischen Antipoden zerlegen, beispielsweise durch Umkristallisation aus einem optisch aktiven Lösungsmittel, mit Hilfe von geeigneten Mikroorganismen oder durch Umsetzen einer Verbindung der Formel I mit salz-bildenden, z.B. basischen, Eigenschaften mit einem optisch aktiven, salzbildenden Mittel, wie einer optisch aktiven Säure, und Trennen der auf diese Weise erhaltenen Gemische von Salzen, z.B. auf Grund ihrer verschiedenen Löslichkeiten, in die diastereomeren Salze, aus denen die Antipoden, z.B. durch Behandeln mit einer Base, freigesetzt werden können.

Optische Antipoden von neutralen Verbindungen der Formel I kann man z.B. auch gemäss Verfahren d) unter Verwendung einer optisch aktiven Säure der Formel VI erhalten, wobei man diese z.B. aus der entsprechenden racemischen Säure in üblicher Weise, z.B. durch Salzbildung mit einer optisch aktiven Base, Trennung der diastereomeren Salze und Freisetzung der optisch aktiven Säure, oder unter Verwenden eines reaktionsfähigen funktionellen Derivats einer optisch aktiven Säure bilden kann.

Ferner kann man z.B. Verbindungen der Formel I, die eine veresterte Carboxygruppe, z.B. eine entsprechende Gruppe Ac, aufweisen, unter Verwendung eines optisch aktiven Alkohols nach dem oben beschriebenen Verfahren umestern und das so erhältliche Diastereomerengemisch, z.B. mittels fraktionierter Kristallisation, in die Antipoden auftrennen.

Vorteilhafterweise isoliert man aus einem Diastereomerengemisch bzw. Racemat das pharmakologisch aktivere Isomere bzw. den aktiveren Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates, z.B. Salzes, und/oder seiner Racemate bzw. Antipoden verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu deren Herstellung bilden ebenfalls einen Gegenstand der vorliegenden Erfindung. Die Erfindung betrifft auch verfahrensgemäss erhältliche neue Zwischenprodukte, sowie Verfahren zu ihrer Herstellung.

Die Erfindung betrifft ebenfalls die Verwendung der Verbindungen der Formel I oder von pharmazeutisch verwendbaren Salzen von solchen Verbindungen mit salzbildenden Eigenschaften, insbesondere als pharmakologisch, in erster Linie als coronardilatatorisch und/oder antihypertensiv verwendbare Verbindungen. Dabei kann man sie, vorzugsweise in Form von pharmazeutischen Präparaten, in einem Verfahren zur prophylaktischen und/oder therapeutischen Behandlung des tierischen oder menschlichen Körpers, insbesondere zur Behandlung von Angina pectoris, Hypertonie und von anderen cardiovasculären Krankheiten verwenden.

Die Dosierung des Wirkstoffs, der allein oder zusammen mit dem üblichen Träger- und Hilfsmaterial verabreicht wird, hängt von der zu behandelnden Spezies, deren Alter und individuellen Zustand, sowie der Verabreichungsweise ab. Die täglichen Dosen liegen für Mammalien mit einem Körpergewicht von etwa 70 kg, je nach Art der Erkrankung, individuellem Zustand und Alter, vorzugsweise zwischen 10 und 300 mg.

Die Erfindung betrifft im weiteren pharmazeutische Präparate, die Verbindungen der Formel I oder pharmazeutisch verwendbare Salze von solchen Verbindungen mit salzbildenden Eigenschaften als Wirkstoffe enthalten, sowie Verfahren zu ihrer Herstellung.

Bei den erfindungsgemässen pharmazeutischen Präparaten handelt es sich um solche zur enteralen, wie peroralen oder rektalen, weiter zur sublingualen, sowie zur parenteralen Verabreichung an Warmblüter. Entsprechende Dosiseinheitsformen, insbesondere zur peroralen und/oder sublingualen Verabreichung, z.B. Dragées, Tabletten oder Kapseln, enthalten vorzugsweise von etwa 2,5 bis etwa 100 mg, insbesondere von etwa 5 bis etwa 50 mg einer Verbindung der Formel I oder eines pharmazeutisch annehmbaren Salzes einer zur Salzbildung befähigten entsprechenden Verbindung zusammen mit pharmazeutisch verwendbaren Trägerstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyäthylenglykol und/oder Titandioxid enthalten, oder Lacklösungen in geeigneten organischen Lösungsmitteln oder Lösungsmittelgemischen, oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können. Bevorzugt sind u.a. Kapseln, die sowohl leicht zer-

bissen werden können, um z.B. bei Anzeichen eines Anfalls von Angina pectoris durch sublinguale Aufnahme des Wirkstoffes eine möglichst rasche Wirkung zu erzielen, als auch unzerkaut geschluckt werden können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässerige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat, oder Triglyceride verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren hergestellt werden. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1: Ein Gemisch von 25,0 g 2-Methyl-4-(2-nitro-phenyl)-5-oxo-1,4,5,7-tetrahydro-furo[3,4-b]pyridin-3-carbonsäure-äthylester und 250 ml einer 33%-igen Lösung von Methylamin in Aethanol wird in einem geschlossenen Rohr während 15 Stunden bei 60° erhitzt. Das Reaktionsgemisch wird unter vermindertem Druck zur Trockne eingedampft und der Rückstand zwischen Methylenchlorid und einer 2-n. wässrigen Natriumcarbonatlösung verteilt. Die organische Phase wird abgetrennt, mit Wasser gewaschen, getrocknet und unter vermindertem Druck eingedampft. Der Rückstand wird in Methanol aufgenommen, mit Aktivkohle behandelt, filtriert und das Filtrat zur Trockne eingedampft. Das zurückbleibende Oel wird aus Essigsäureäthylester kristallisiert und man erhält so den 2-Hydroxymethyl-6-methyl-3-methylaminocarbonyl-4-(2-nitro-phenyl)-1,4-dihydropyridin-5-carbonsäure-äthylester, der bei 172-173° schmilzt.

Das Ausgangsmaterial kann wie folgt erhalten werden: Ein Gemisch von 45,2 g 2-Nitrobenzaldehyd, 38,8 g 3-Amino-crotonsäureäthylester, und 49,4 g 4-Chloracetessigsäureäthylester in 200 ml absolutem Aethanol während 30 Stunden auf 60° erhitzt. Die Reaktionslösung wird in einem Eisbad gekühlt, wobei das Produkt kristallisiert; es wird abfiltriert und der Filterrückstand wird aus Aethanol umkristallisiert. Der so erhältliche 2-Methyl-4-(2-nitro-phenyl)-5-oxo-1,4,5,7-tetrahydro-furo[3,4-b]pyridin-3-carbonsäure-äthylester schmilzt bei 215-217°.

Beispiel 2: Analog dem im Beispiel 1 beschriebenen Verfahren erhält man aus einem Gemisch von 12,0 g 2-Methyl-4-(3-nitro-phenyl)-5-oxo-1,4,5,7-tetrahydro-furo[3,4-b]pyridin-3-carbonsäure-äthylester und 120 ml einer 33%igen Lösung von Methylamin in Aethanol den 2-Hydroxymethyl-6-methyl-3-methylaminocarbonyl-4-(3-nitro-phenyl)-1,4-dihydropyridin-5-carbonsäure-äthylester, der nach Umkristallisation aus Isopropanol bei 168-169° schmilzt.

Das Ausgangsmaterial kann wie folgt erhalten werden: Analog dem im Beispiel 1 beschriebenen Verfahren erhält man aus einem Gemisch von 45,2 g 3-Nitrobenzaldehyd, 38,8 g 3-Amino-crotonsäureäthylester und 49,4 g 4-Chlor-acetessigsäureäthylester in 200 ml absolutem Aethanol den 2-Methyl-4-(3-nitro-phenyl)-5-oxo-1,4,5,7-tetrahydro-furo[3,4-b]-pyridin-3-carbonsäure-äthylester, der nach Umkristallisieren aus Essigsäureäthylester bei 175-180° schmilzt.

Beispiel 3: Analog dem im Beispiel 1 beschriebenen Verfahren erhält man aus einem Gemisch von 13,5 g 2-Methyl-5-oxo-4-(2,3,4,5,6-pentafluor-phenyl)-1,4,5,7-tetrahydro-furo[3,4-b]pyridin-3-carbonsäure-äthylester und 135 ml einer 33%igen Lösung von Methylamin in Aethanol den 2-Hydroxymethyl-6-methyl-3-methylaminocarbonyl-4-(4-methylamino-2,3,5,6-tetrafluor-phenyl)-1,4-dihydro-pyridin-5-carbonsäure-äthylester, der nach Umkristallisieren aus Isopropanol bei 176-177° schmilzt.

Das Ausgangsmaterial kann wie folgt erhalten werden: Analog dem im Beispiel 1 beschriebenen Verfahren erhält man aus einem Gemisch von 12,0 g 2,3,4,5,6-Pentafluorbenzaldehyd, 7,7 g 3-Amino-crotonsäure-äthylester und 10,0 g 4-Chloracetessigsäureäthylester in 60 ml absolutem Aethanol den 2-Methyl-5-oxo-4-(2,3,4,5,6-pentafluor-phenyl)-1,4,5,7-tetrahydro-furo[3,4-b]pyridin-3-carbonsäure-äthylester, der nach Umkristallisieren aus Isopropanol bei 248-251° schmilzt.

Beispiel 4: Analog dem im Beispiel 1 beschriebenen Verfahren erhält man aus einem Gemisch von 14,7 g 4-(3-Cyan-phenyl)-2-methyl-5-oxo-1,4,5,7-tetrahydro-furo[3,4-b]pyridin-3-carbonsäure-äthylester und 147 ml einer 33%igen Lösung von Methylamin in Aethanol den 4-(3-Cyan-phenyl)-2-hydroxymethyl-6-methyl-3-methylaminocarbonyl-1,4-dihydro-pyridin-5-carbonsäure-äthylester, der nach Umkristallisieren aus einem Gemisch von Isopropanol und Petroläther bei 173-175° schmilzt.

Das Ausgangsmaterial kann wie folgt erhalten werden: Analog dem im Beispiel 1 beschriebenen Verfahren erhält man aus einem Gemisch von 19,7 g 3-Cyanbenzaldehyd, 19,4 g 3-Amino-crotonsäureäthylester und 24,7 g 4-Chlor-acetessigsäureäthylester in 600 ml absolutem Aethanol den 4-(3-Cyan-phenyl)-2-methyl-5-oxo-1,4,5,7-tetrahydro-furo[3,4-b]-pyridin-3-carbonsäure-äthylester, der nach Umkristallisieren aus Isopropanol bei 186-189° schmilzt.

Beispiel 5: Analog dem im Beispiel 1 beschriebenen Verfahren erhält man aus einem Gemisch von 15,0 g 4-(2,3-Dichlor-phenyl)-2-methyl-5-oxo-1,4,5,7-tetrahydro-furo[3,4-b]pyridin-3-carbonsäure-äthylester und 150 ml einer 33%igen Lösung von Methylamin in Aethanol den 4-(2,3-Dichlor-phenyl)-2-hydroxymethyl-6-methyl-3-methylaminocarbonyl-1,4-dihydro-pyridin-5-carbonsäure-äthylester, der nach Umkristallisieren aus einem Gemisch von Isopropanol und Diäthyläther bei 215-217° schmilzt.

Das Ausgangsmaterial kann analog dem im Beispiel 1 beschriebenen Verfahren aus einem Gemisch von 16,3 g 2,3-Dichlorbenzaldehyd, 12,0 g 3-Amino-crotonsäureäthylester und 15,3 g 4-Chlor-acetessigsäureäthylester in 280 ml absolutem Aethanol erhalten werden; der 4-(2,3-Dichlor-phenyl)-2-methyl-5-oxo-1,4,5,7-tetrahydro-furo[3,4-b]pyridin-3-carbonsäure-äthylester schmilzt nach Umkristallisieren aus einem Gemisch von Methanol, Chloroform und Diäthyläther bei 255-258°.

Beispiel 6: Analog dem im Beispiel 1 beschriebenen Verfahren erhält man aus einem Gemisch von 13,0 g 2-Methyl-5-oxo-4-(2-trifluormethyl-phenyl)-1,4,5,7-tetrahydro-furo[3,4-b]pyridin-3-carbonsäure-äthylester und 130 ml einer 33%igen Lösung von Methylamin in Aethanol den 2-Hydroxymethyl-6-methyl-3-methylaminocarbonyl-4-(2-trifluormethyl-phenyl)-1,4-dihydro-pyridin-5-carbonsäure-äthylester, der bei 214-216° schmilzt.

Das Ausgangsmaterial kann wie folgt erhalten werden: Analog dem im Beispiel 1 beschriebenen Verfahren erhält man aus einem Gemisch von 32,5 g 2-Trifluormethyl-benzaldehyd, 24,1 g 3-Amino-crotonsäureäthylester und 30,8 g 4-Chlor-acetessigsäureäthylester in 500 ml absolutem Aethanol den 2-Methyl-5-oxo-4-(2-trifluormethyl-phenyl)-1,4,5,7-tetrahydro-furo[3,4-b]pyridin-3-carbonsäure-äthylester, der nach Umkristallisieren aus einem Gemisch von Isopropanol und Petroläther bei 203-205° schmilzt.

Beispiel 7: Ein Gemisch von 7,4 g 4-(3,4-Dichlor-2-thienyl)-2-methyl-5-oxo-1,4,5,7-tetrahydro-furo[3,4-b]pyridin-3-carbonsäureäthylester und 75 ml einer 33%igen Lösung von Methylamin in Aethanol wird im geschlossenen Rohr während 15 Stunden auf 60° erhitzt, das Reaktionsgemisch anschliessend unter vermindertem Druck zur Trockne eingedampft und der Rückstand zwischen Methylenchlorid und 2-n wässriger Natriumcarbonatlösung verteilt, die organische Phase abgetrennt, mit Wasser gewaschen, über Natriumsulfat getrocknet und unter vermindertem Druck eingedampft. Der Rückstand wird aus Methanol umkristallisiert, wonach man den 4-(3,4-Dichlor-2-thienyl)-6-hydroxymethyl-2-methyl-5-methylaminocarbonyl-1,4-dihydro-pyridin-3-carbonsäure-äthylester erhält, welcher bei 217-219° schmilzt.

Das Ausgangsmaterial kann wie folgt erhalten werden: Ein Gemisch von 6,1 g 3,4-Dichlorthiophen-carboxaldehyd, 4,3 g 3-Aminocrotonsäure-äthylester und 5,5 g 4-Chloracetessigsäureäthylester in 120 ml

absolutem Aethanol wird während 30 Stunden auf 60° erhitzt.

Nach dem Abkühlen des Reaktionsgemisches im Eisbad fallen Kristalle aus, die abgesaugt und aus Aethanol umkristallisiert werden. Der so erhaltene 4-(3,4-Dichlor-2-thienyl)-2-methyl-5-oxo-1,4,5,6-tetrahydro-furo[3,4-b]pyridin-3-carbonsäure-äthylester schmilzt bei 210-215°.

Beispiel 8: Zu einer Lösung von 3,73 g 2-Formyl-6-methyl-3-methylaminocarbonyl-4-(2-nitrophenyl)-1,4-dihydro-pyridin-5-carbonsäure-äthylester in 60 ml Aethanol werden unter Rühren bei 0-5° 430 mg Natriumborhydrid in kleinen Portionen zugegeben, und anschliessend bei Raumtemperatur noch 2 Stunden gerührt. Dann wird mit 2-n Salzsäure angesäuert und unter vermindertem Druck zur Trockne eingedampft. Der Rückstand wird zwischen Wasser und Essigsäureäthylester verteilt, die organische Phase über Magnesiumsulfat getrocknet und zur Trockne verdampft. Das zurückbleibende Oel wird aus Essigsäureäthylester kristallisiert, wonach man den 2-Hydroxymethyl-6-methyl-3-methylaminocarbonyl-4-(2-nitrophenyl)-1,4-dihydro-pyridin-5-carbonsäure-äthylester erhält, der bei 172-173° schmilzt.

Das Ausgangsprodukt kann wie folgt erhalten werden: Zu einem Gemisch von 101,2 g Diisopropylamin in 1000 ml Tetrahydrofuran werden bei -20° 600 ml n-Butyllithium in Hexan langsam zugetropft und 20 Minuten ausgerührt. Dann wird in 10 Minuten bei -10° ein Gemisch von 36,5 g N-Methylacetamid und 50 ml Tetrahydrofuran zugegeben und 10 Minuten weitergerührt. Hierauf werden 88,0 g Diäthoxyessigsäureäthylester bei Raumtemperatur zugetropft und 1 Stunde bei dieser Temperatur belassen. Die Reaktionslösung wird bei 0-5° zunächst mit 10 ml Wasser und anschliessend mit einem Gemisch von 100 ml Eisessig und 100 ml Wasser zersetzt. Die Phasen werden getrennt und die wässrige Phase mit Aether ausgezogen. Die vereinigten organischen Phasen werden mit Natriumbicarbonatlösung, dann mit Wasser gewaschen, über Natriumsulfat getrocknet und verdampft. Das zurückbleibende 4-Diäthoxyacet-

essigsäuremethylamid wird bei 105-110°/0,1 mm destilliert.

Ein Gemisch von 40,6 g des erhaltenen Produktes, 30,2 g 2-Nitrobenzaldehyd und 1 ml Piperidin wird in 300 ml Toluol 15 Stunden am Wasserabscheider erhitzt, die Reaktionslösung unter vermindertem Druck eingedampft, wonach man 2-(2-Nitrophenyl-methylen)-4-diäthoxy-acetessigsäuremethylamid als Rückstand erhält. Ein Gemisch von 33,6 g des erhaltenen Rohprodukts und 25,8 g 3-Amino-crotonsäureäthylester wird 12 Stunden auf 110-120° erhitzt, das erhaltene schwarze Oel zwischen Essigsäureäthylester und Wasser verteilt, die organische Phase über Magnesiumsulfat getrocknet und verdampft, wonach man 2-Diäthoxymethyl-6-methyl-3-methylaminocarbonyl-4-(2-nitrophenyl)-1,4-dihydropyridin-5-carbonsäure-äthylester als Rohprodukt erhält.

22,35 g der erhaltenen Verbindung werden in 220 ml Aceton mit 20 ml 6-n. Salzsäure 8 Stunden bei Raumtemperatur gerührt. Das Reaktionsgemisch wird unter vermindertem Druck eingedampft, der Rückstand in Methylenchlorid aufgenommen, die organische Phase mit Natriumbicarbonatlösung und Wasser gewaschen, über Magnesiumsulfat getrocknet und eingedampft. Der zurückbleibende 2-Formyl-6-methyl-3-methylaminocarbonyl-4-(2-nitrophenyl)-1,4-dihydro-pyridin-5-carbonsäureäthylester wird als solcher weiter verarbeitet.

Beispiel 9: Ein Gemisch von 1,8 g 3-Carboxy-2-hydroxymethyl-6-methyl-4-(2-nitrophenyl)-1,4-dihydro-pyridin-5-carbonsäure-äthylester und 20 ml Oxalylchlorid wird 1 Stunde unter Rückfluss erhitzt. Das Reaktionsgemisch wird dann unter vermindertem Druck zur Trockne eingedampft und der Rückstand durch Abdestillieren mit Toluol von flüchtigen Anteilen befreit. Das zurückbleibende Oel wird nun mit 20 ml einer 33%igen Lösung von Methylamin in Aethanol versetzt und während 2 Stunden unter Rückfluss erhitzt. Die Reaktionslösung wird eingedampft, der Rückstand zwischen Essigsäureäthylester und Wasser verteilt, die organische Phase über Natriumsulfat getrocknet und verdampft. Der so erhaltene 6-Hydroxymethyl-2-methyl-5-methylamino-

carbonyl-4-(2-nitrophenyl)-1,4-dihydro-pyridin-3-carbonsäure-äthylester schmilzt nach Umkristallisieren aus Essigsäureäthylester bei 172-173°.

Das Ausgangsmaterial kann wie folgt erhalten werden: Ein Gemisch von 2,75 g 2-Methyl-4-(2-nitrophenyl)-5-oxo-1,4,5,7-tetrahydro-furo[3,4-b] pyridin-3-carbonsäure-äthylester und 16 ml 2-n. Kalilauge wird 2 1/2 Stunden bei 100° gerührt. Die schwarze Reaktionslösung wird mit 6-n. Salzsäure sauer gestellt und unter vermindertem Druck zur Trockne eingedampft. Der Rückstand wird durch Abdestillieren mit Toluol getrocknet, in kaltem Methanol aufgenommen, von Unlöslichem abfiltriert und das Filtrat zur Trockne verdampft. Der Rückstand wird in kaltem Isopropanol gelöst, von Unlöslichem befreit und das Filtrat eingedampft. Der zurückbleibende rohe 3-Carboxy-2-hydroxymethyl-6-methyl-4-(2-nitrophenyl)-1,4-dihydro-pyridin-5-carbonsäure-äthylester wird als solcher weiter verarbeitet.

<u>Beispiel 10</u>: Ein Gemisch von 3,28 g 2-Hydroxymethyl-6-methyl-3-methylaminocarbonyl-4-(2-nitrophenyl)-1,4-dihydro-pyridin-3-carbonitril, 100 ml absolutem Aethanol und 0,18 g Wasser wird während 2 1/2 Stunden unter Einleiten eines kräftigen Stroms Chlorwasserstoff auf 70° erhitzt. Anschliessend wird das Gemisch unter vermindertem Druck zur Trockne eingedampft und der Rückstand zwischen Eiswasser und Essigsäureäthylester verteilt, die organische Phase mit Natriumbicarbonatlösung und wässriger Natriumchloridlösung gewaschen, über Natriumsulfat getrocknet und eingedampft. Das zurückbleibende Oel wird aus Essigsäureäthylester kristallisiert, wonach man den 6-Hydroxymethyl-2-methyl-5-methylaminocarbonyl-4-(2-nitrophenyl)-1,4-dihydro-pyridin-3-carbonsäure-äthylester erhält, der bei 172-173° schmilzt.

Das Ausgangsmaterial kann wie folgt erhalten werden: Ein Gemisch von 10,0 g 2-Nitrobenzaldehyd, 5,4 g 3-Amino-crotonsäurenitril und 10,9 g 4-Chlor-cetessigsäureäthylester in 100 ml absolutem Aethanol wird während 76 Stunden unter Rückfluss erhitzt. Die Reaktionslösung wird

abgekühlt und die ausgefallenen Kristalle abfiltriert. Diese werden in viel Essigsäureäthylester gelöst, die Lösung mehrmals mit Wasser ausgezogen, die organische Phase über Natriumsulfat getrocknet, und unter vermindertem Druck eingedampft. Das so erhaltene 2-Methyl-4-(2-nitrophenyl)-5-oxo-1,4,5,6-tetrahydro-furo[3,4-b]pyridin-3-carbonitril wird aus einem Gemisch von Aceton und Petroläther umkristallisiert; es schmilzt bei 265-266°.

Ein Gemisch von 5,94 g des obigen Produktes und 60 ml einer 33%igen Lösung von Methylamin in Aethanol wird in einem geschlossenen Rohr während 15 Stunden auf 60° erhitzt. Das Reaktionsgemisch wird unter vermindertem Druck zur Trockne eingedampft, und der Rückstand zwischen Methylenchlorid und 2-n. wässriger Natriumcarbonatlösung verteilt. Die organische Phase wird abgetrennt, mit Wasser gewaschen, über Natriumsulfat getrocknet und unter vermindertem Druck eingedampft, wonach man das 2-Hydroxymethyl-6-methyl-3-methylaminocarbonyl-4-(2-nitrophenyl)-1,4-dihydro-pyridin-3-carbonitril erhält, welches als Rohprodukt weiter verarbeitet wird.

Beispiel 11: Ein Gemisch von 26,4 g 4-Hydroxy-2-(2-nitrophenylmethylen)-acetessigsäure-methylamid, 12,9 g 3-Amino-crotonsäureäthylester in 500 ml absolutem Aethanol wird während 16 Stunden unter Rückfluss erhitzt, das Gemisch unter vermindertem Druck zur Trockne eingedampft und der Rückstand zwischen Essigsäureäthylester und Wasser verteilt. Die organische Phase wird mit Natriumsulfat getrocknet, eingedampft und der Rückstand aus Essigsäureäthylester umkristallisiert. Der so erhaltene 2-Hydroxymethyl-6-methyl-3-methylaminocarbonyl-4-(2-nitrophenyl)-1,4-dihydro-pyridin-5-carbonsäureäthylester schmilzt bei 172-173°.

Das Ausgangsprodukt kann wie folgt erhalten werden: Ein Gemisch von 40,6 g 4-Diäthoxy-acetessigsäure-methylamid, 400 ml Aceton und 40 ml 6-n. Salzsäure wird bei Raumtemperatur 8 Stunden gerührt, die Reaktionslösung unter vermindertem Druck eingedampft, der Rückstand in Methylenchlorid aufgenommen, die organische Phase mit wässriger

Natriumbicarbonatlösung, dann mit Wasser gewaschen, getrocknet und erneut eingedampft. Das so erhaltene Glyoxyl-methylacetamid wird als solches weiter verarbeitet.
Zu einer Suspension von 7,56 g Natriumborhydrid in 80 ml Benzol werden 39,0 g Eisessig zugegeben und das Gemisch während 15 Minuten unter Rückfluss erhitzt. Zu der so erhaltenen Lösung von Triacetoxyborhydrid werden 12,9 g Glyoxyl-methylacetamid gegeben und das Gemisch 1 Stunde unter Rückfluss erhitzt. Nach dem Abkühlen wird das Reaktionsgemisch mit Wasser versetzt, die organische Phase abgetrennt, über Natriumsulfat getrocknet und verdampft. Das so erhaltene 4-Hydroxy-acetessigsäure-methylamid wird roh weiter verarbeitet.

Ein Gemisch von 13,1 g der erhaltenen rohen Verbindung, 15,1 g 2-Nitrobenzaldehyd und 1 ml Piperidin wird in 400 ml absolutem Benzol während 16 Stunden unter Abscheidung von entstandenem Wasser unter Rückfluss erhitzt. Die Reaktionslösung wird anschliessend unter vermindertem Druck eingedampft und das erhaltene 4-Hydroxy-2-(2-nitrophenyl-methylen)-acetessigsäure-methylamid roh weiter verarbeitet.

Beispiel 12: Ein Gemisch von 13,1 g 4-Hydroxy-acetessigsäure-methylamid, 15,1 g 2-Nitrobenzaldehyd und 12,9 g 3-Amino-crotonsäure-äthylester in 500 ml absolutem Aethanol wird 15 Stunden unter Rückfluss erhitzt. Anschliessend wird das Reaktionsgemisch unter vermindertem Druck zur Trockne eingedampft und der Rückstand zwischen Essigsäureäthylester und Wasser verteilt. Die organische Phase wird über Natriumsulfat getrocknet, verdampft und der Rückstand aus Essigsäureäthylester umkristallisiert. Man erhält so den 2-Hydroxymethyl-6-methyl-3-methylaminocarbonyl-4-(2-nitrophenyl)-1,4-dihydro-pyridin-5-carbonsäure-äthylester, der bei 172-173° schmilzt.

Beispiel 13: Analog dem im Beispiel 1 beschriebenen Verfahren erhält man aus einem Gemisch von 14,45 g 4-(2-Furyl)-2-methyl-5-oxo-1,4,5,7-tetrahydro-furo[3,4-b]pyridin-3-carbonsäure-äthylester und 150 ml einer 33%igen Lösung von Methylamin in Aethanol den 2-Hydroxymethyl-6-methyl-3-methylaminocarbonyl-4-(2-furyl)-1,4-dihydro-pyridin-5-carbonsäure-äthylester, der durch folgende spektroskopische Kenndaten charakterisiert wird:

$^1$H-NMR. (250 MHz-FT, $CDCl_3$): 1,30 (t, 3H, $-COO-C-CH_3$); 2,30 (s, 3H, $-CH_3$); 2,75 (d, 3H, $-NH-CH_3$); 4,15 (m, 2H, $-COO-CH_2-C$); 4,25 (t, 1H, -CO-OH); 4,65 (d, 2H, $-CH_2-O$); 5,2 (s, 1H, C-H(4)); 5,95, 6,2, 7,2 (m, 3H, Furan); 7,05 (s, 1H, NH); 7,3 (s, 1H, -CO-NH-C).

Das Ausgangsmaterial kann wie folgt erhalten werden: Analog Beispiel 1 erhält man aus einem Gemisch von 28,8 g Furan-2-carboxaldehyd, 38,8 g 3-Amino-crotonsäureäthylester und 33,6 g 4-Chloracetessigsäureäthylester in 200 ml absolutem Aethanol den 4-(2-Furyl)-2-methyl-5-oxo-1,4,5,7-tetrahydro-furo[3,4-b]pyridin-3-carbonsäure-äthylester, der nach Umkristallisieren aus Essigsäureäthylester bei 236-237° schmilzt.

Beispiel 14: Analog dem im Beispiel 1 beschriebenen Verfahren erhält man aus einem Gemisch von 4 g 2-Methyl-3-methylsulfonyl-4-(2-nitro-phenyl)-5-oxo-1,4,5,7-tetrahydro-furo[3,4-b]pyridin und 100 ml einer 33%igen Lösung von Methylamin in Aethanol das 2-Hydroxymethyl-6-methyl-3-methylaminocarbonyl-5-methylsulfonyl-4-(2-nitro-phenyl)-1,4-dihydro-pyridin, das nach Umkristallisation aus einem Gemisch von Methylenchlorid und Hexan bei 218-219° unter Zersetzung schmilzt.

Das Ausgangsmaterial kann wie folgt erhalten werden: Ein Gemisch von 2,0 g 3-Methylsulfonyl-4-(2-nitro-phenyl)-3-buten-2-on und 0,74 g 4-Amino-2,5-dihydro-furan-2-on wird während 14 Stunden auf 130° erhitzt. Das erstarrte Rohprodukt wird aus einem Gemisch von Aceton, Diisopropyläther und Aether umkristallisiert und die Kristalle werden bei 80° am Hochvakuum getrocknet. Das so erhältliche 2-Methyl-3-methylsulfonyl-4-(2-nitrophenyl)-5-oxo-1,4,5,7-tetrahydro-furo[3,4-b]pyridin schmilzt bei 165-167°.

Beispiel 15: Analog den in der Beschreibung beschriebenen und den in den Beispielen illustrierten Verfahren können, ausgehend von entsprechenden Ausgangsstoffen, u.a. folgende Verbindungen der Formel I oder deren Salze hergestellt werden:

a) 4-(2-Chlorphenyl)-2-hydroxymethyl-6-methyl-3-methylaminocarbonyl-1,4-dihydro-pyridin-5-carbonsäure-äthylester durch Behandeln von 4-(2-Chlorphenyl)-2-methyl-5-oxo-1,4,5,7-tetrahydro-furo[3,4-b]pyridin-3-carbonsäure-äthylester mit Methylamin;

b) 4-(2-Cyan-phenyl)-2-hydroxymethyl-6-methyl-3-methylaminocarbonyl-1,4-dihydro-pyridin-5-carbonsäure-äthylester durch Behandeln von 4-(2-Cyanphenyl)-2-methyl-5-oxo-1,4,5,7-tetrahydro-furo[3,4-b]pyridin-3-carbonsäure-äthylester mit Methylamin;

c) 2-Hydroxymethyl-6-methyl-3-methylaminocarbonyl-4-(3-trifluormethyl-phenyl)-1,4-dihydro-pyridin-5-carbonsäure-äthylester durch Behandeln von 2-Methyl-5-oxo-4-(3-trifluormethyl-phenyl)-1,4,5,7-tetrahydro-furo[3,4-b]pyridin-3-carbonsäure-äthylester mit Methylamin;

d) 2-Hydroxymethyl-6-methyl-3-methylaminocarbonyl-4-(2-pyridyl)-1,4-dihydro-pyridin-5-carbonsäure-äthylester durch Behandeln von 2-Methyl-5-oxo-4-(2-pyridyl)-1,4,5,7-tetrahydro-furo]3,4-b]pyridin-3-carbonsäure-äthylester mit Methylamin;

e) 4-(2-Chlor-3-pyridyl)-2-hydroxymethyl-6-methyl-3-methylaminocarbonyl-1,4-dihydro-pyridin-5-carbonsäure-äthylester durch Behandeln von 4-(2-Chlor-3-pyridyl)-2-methyl-5-oxo-1,4,5,7-tetrahydro-furo[3,4-b]-pyridin-3-carbonsäure-äthylester mit Methylamin;

f) 4-(2,3-Dimethyl-phenyl)-2-hydroxymethyl-6-methyl-3-methylaminocarbonyl-1,4-dihydro-pyridin-5-carbonsäure-äthylester, der bei 182-183° schmilzt, durch Behandeln von 4-(2,3-Dimethyl-phenyl)-2-methyl-5-oxo-1,4,5,7-tetrahydro-furo[3,4-b]pyridin-3-carbonsäure-äthylester mit Methylamin;

g) 2-Hydroxymethyl-6-methyl-3-methylaminocarbonyl-4-(2-methylthio-3-pyridyl)-1,4-dihydro-pyridin-5-carbonsäure-äthylester durch Behandeln von 2-Methyl-5-oxo-4-(2-methylthio-3-pyridyl)-1,4,5,7-tetrahydro-furo[3,4-b]pyridin-3-carbonsäure-äthylester mit Methylamin;

h) 4-(4-Chlor-2-thienyl)-2-hydroxymethyl-6-methyl-3-methylaminocarbonyl-1,4-dihydro-pyridin-5-carbonsäure-äthylester durch Behandeln von 4-(4-Chlor-2-thienyl)-2-methyl-5-oxo-1,4,5,7-tetrahydro-furo[3,4-b] pyridin-3-carbonsäure-äthylester mit Methylamin;

i) 3-Aethylaminocarbonyl-2-hydroxymethyl-6-methyl-4-(2-nitro-phenyl)-1,4-dihydro-pyridin-5-carbonsäure-äthylester, der bei 158-160° schmilzt, durch Behandeln von 2-Methyl-4-(2-nitro-phenyl)-5-oxo-1,4,5,7-tetrahydro-furo[3,4-b]pyridin-3-carbonsäure-äthylester mit Aethylamin;

j) 3-Aethylaminocarbonyl-2-hydroxymethyl-6-methyl-4-(2-nitro-phenyl)-1,4-dihydro-pyridin-5-carbonsäure-methylester, der bei 195-198° schmilzt, durch Behandeln von 2-Methyl-4-(2-nitro-phenyl)-5-oxo-1,4,5,7-tetrahydro-furo[3,4-b]pyridin-3-carbonsäure-methylester mit Aethylamin;

k) 2-Hydroxymethyl-6-methyl-3-methylaminocarbonyl-4-(2-nitro-phenyl)-1,4-dihydro-pyridin-5-carbonsäure-methylester, der bei 191-193° schmilzt, durch Behandeln von 2-Methyl-4-(2-nitro-phenyl)-5-oxo-1,4,5,7-tetrahydro-furo[3,4-b]pyridin-3-carbonsäure-methylester mit Methylamin;

l) 2-Hydroxymethyl-6-methyl-3-methylaminocarbonyl-4-(2-nitro-phenyl)-1,4-dihydropyridin-5-carbonsäure-isopropylester durch Behandeln von 2-Methyl-4-(2-nitro-phenyl)-5-oxo-1,4,5,7-tetrahydro-furo[3,4-b]pyridin-3-carbonsäure-isopropylester mit Methylamin;

m) 2-Hydroxymethyl-3-isopropylaminocarbonyl-6-methyl-4-(3-nitro-phenyl)-1,4-dihydro-pyridin-5-carbonsäure-(2-methoxyäthyl)-ester durch Behandeln von 2-Methyl-4-(3-nitro-phenyl)-5-oxo-1,4,5,7-tetrahydro-furo[3,4-b]pyridin-3-carbonsäure-(2-methoxyäthyl)-ester mit Isopropylamin;

n) 2-Hydroxymethyl-6-methyl-3-methylaminocarbonyl-4-(3-nitro-phenyl)-1,4-dihydro-pyridin-5-carbonsäure-[2-(N-benzyl-N-methyl-amino)-äthyl]-ester durch Behandeln von 2-Methyl-4-(3-nitro-phenyl)-5-oxo-1,4,5,7-tetrahydro-furo[3,4-b]pyridin-3-carbonsäure-[2-(N-benzyl-N-methyl-amino)-äthyl]-ester mit Methylamin;

o) 2-Hydroxymethyl-6-methyl-3-methylaminocarbonyl-1-[2-(4-morpholino)-äthyl]-4-(2-nitro-phenyl)-1,4-dihydro-pyridin-5-carbonsäure-äthylester durch Behandeln von 2-Hydroxymethyl-6-methyl-3-methylaminocarbonyl-4-(2-nitro-phenyl)-1,4-dihydro-pyridin-5-carbonsäure-äthylester mit 2-(4-Morpholino)-äthyljodid und Natriumhydrid in Dimethylformamid;

p) 2-Hydroxymethyl-6-methyl-3-methylaminocarbonyl-4-(2-nitro-phenyl)-1,4-dihydro-pyridin-5-carbonsäure-isobutylester, der bei 176-177° schmilzt, durch Behandeln von 2-Methyl-4-(2-nitro-phenyl)-5-oxo-1,4,5,7-tetrahydro-furo[3,4-b]pyridin-3-carbonsäure-isobutylester mit Methylamin;

q) 2-Hydroxymethyl-6-methyl-3-methylaminocarbonyl-4-(2-nitro-phenyl)-1,4-dihydro-pyridin-5-carbonsäure-(2-methoxyäthyl)-ester, der bei 152-154° schmilzt, durch Behandeln von 2-Methyl-4-(2-nitro-phenyl)-5-oxo-1,4,5,7-tetrahydro-furo[3,4-b]pyridin-3-carbonsäure-(2-methoxyäthyl)-ester mit Methylamin;

r) 2-Hydroxymethyl-6-methyl-3-methylaminocarbonyl-4-(2-difluormethoxy-phenyl)-1,4-dihydro-pyridin-5-carbonsäure-äthylester, der bei 161-164° schmilzt, durch Behandeln von 2-Methyl-4-(2-difluormethoxy-phenyl)-5-oxo-1,4,5,7-tetrahydro-furo[3,4-b]pyridin-3-carbonsäure-äthylester mit Methylamin;

s) 2-Hydroxymethyl-6-methyl-3-(2-dimethylamino-äthyl)-aminocarbonyl-4-(2-nitro-phenyl)-1,4-dihydro-pyridin-5-carbonsäure-äthylester, durch Behandeln von 2-Methyl-4-(2-nitro-phenyl)-5-oxo-1,4,5,6-tetrahydro-furo[3,4-b]pyridin-3-carbonsäure-äthylester mit (2-Dimethylamino-äthyl)-amin im geschlossenen Rohr während 17 Stunden bei 100°.
Spektroskopische Kenndaten:
$^1$H-NMR (100 MHz, $CDCl_3$):1,30 (t, 3H, $-COO-C-CH_3$); 2,30 (s, 3H, $-CH_3$); 2,35 (s, 6H, $-NCCH_3)_2$); 2,78 (t, 2H, $-N-CH_2$); 3,25 (t, 2H, $-CO-n-CH_2$); 4,15 (m, 2H, $-COO-CH_2-C$); 4,25 (t, 1H, -C-OH); 4,70 (s, 2H, $-CH_2-O$); 5,65 (s, 1H, C-H(a)); 7,2-7,9 (m, 6H, Aromat + CO-NH-C + NH).

t) 2-Hydroxymethyl-6-methyl-3-methylaminocarbonyl-4-[2-(2-methoxyäthoxy)-phenyl]-1,4-dihydropyridin-5-carbonsäure-äthylester durch Behandeln von 2-Methyl-4-[2-(2-methoxyäthoxy)-phenyl]-5-oxo-1,4,5,7-tetrahydro-furo[3,4-b]pyridin-3-carbonsäure-äthylester mit Methylamin,

u) 2-Hydroxymethyl-6-methyl-3-methylaminocarbonyl-4-[2-(2-methylthioäthoxy)-phenyl]-1,4-dihydro-pyridin-5-carbonsäure-äthylester, der bei 53-57° schmilzt, durch Behandeln von 2-Methyl-4-[2-(2-methylthioäthoxy)-phenyl]-5-oxo-1,4,5,7-tetrahydro-furo[3,4-b]pyridin-3-carbonsäureäthylester mit Methylamin.

Beispiel 16: Tabletten, enthaltend 25 mg 2-Hydroxymethyl-6-methyl-3-methylaminocarbonyl-4-(2-nitro-phenyl)-1,4-dihydro-pyridin-5-carbonsäureäthylester, können wie folgt hergestellt werden:

Zusammensetzung: (für 1000 Tabletten)

| | |
|---|---|
| 2-Hydroxymethyl-6-methyl-3-methylaminocarbonyl-4-(2-nitro-phenyl)-1,4-dihydro-pyridin-5-carbonsäureäthylester | 25.0 g |
| Maisstärke | 70.0 g |
| Lactose (fein) | 78.5 g |
| Cellulose | 75.0 g |
| Magnesiumstearat | 1.5 g |
| Wasser | q.s. |

Der 2-Hydroxymethyl-6-methyl-3-methylaminocarbonyl-4-(2-nitro-phenyl)-1,4-dihydro-pyridin-5-carbonsäureäthyläther wird mit 60 g Maisstärke und der Lactose vermischt und mit einem aus 10 g Maisstärke und Wasser hergestellten Kleister geknetet. Die feuchte Masse wird granuliert, getrocknet und mit der kristallinen Cellulose und dem Magnesiumstearat gemischt. Die homogene Mischung wird zu Tabletten von 250 mg (mit Bruchrille) verpresst; diese haben einen Durchmesser von 9 mm.

Beispiel 17: Kapseln enthaltend 10 mg an aktiver Substanz können wie folgt hergestellt werden:

Zusammensetzung:

| | |
|---|---|
| 2-Hydroxymethyl-6-methyl-3-methylaminocarbonyl-4-(2-nitro-phenyl)-1,4-dihydro-pyridin-5-carbonsäure-äthylester | 2500 mg |
| Talkum | 200 mg |
| Kolloidale Kieselsäure | 50 mg |

Herstellung:

Die aktive Substanz wird mit Talkum und kolloidaler Kieselsäure innig gemischt, das Gemisch durch ein Sieb mit 0,5 mm Maschenweite getrieben

und dieses in Portionen von jeweils 11 mg in Hartgelatinekapseln geeigneter Grösse abgefüllt.

Beispiel 18: Eine sterile Lösung von 5,0 g 2-Hydroxymethyl-6-methyl-3-methylaminocarbonyl-4-(2-nitro-phenyl)-1,4-dihydro-pyridin-5-carbonsäure-äthylester in 5000 ml destilliertem Wasser wird in Ampullen zu 5 ml abgefüllt, die in 5 ml Lösung 5 mg Wirkstoff enthalten.

Beispiel 19: Anstelle der in den Beispielen 16-18 als Wirkstoff verwendeten Verbindungen können auch folgende Verbindungen der Formel I oder, sofern sie salzbildende Eigenschaften aufweisen, deren pharmazeutisch verwendbare Säureadditionssalze als Wirkstoffe in Tabletten, Dragées, Kapseln, Ampullenlösungen etc. verwendet werden:

2-Hydroxymethyl-6-methyl-3-methylaminocarbonyl-4-(3-nitro-phenyl)-1,4-dihydro-pyridin-5-carbonsäure-äthylester,
2-Hydroxymethyl-6-methyl-3-methylaminocarbonyl-4-(4-methylamino-2,3,5,6-tetrafluor-phenyl)-1,4-dihydro-pyridin-5-carbonsäure-äthylester,
4-(3-Cyanphenyl)-2-hydroxymethyl-6-methyl-3-methylaminocarbonyl-1,4-dihydro-pyridin-5-carbonsäure-äthylester,
4-(2,3-Dichlor-phenyl)-2-hydroxymethyl-6-methyl-3-methylaminocarbonyl-1,4-dihydro-pyridin-5-carbonsäure-äthylester,
2-Hydroxymethyl-6-methyl-3-methylaminocarbonyl-4-(2-trifluormethyl-phenyl)-1,4-dihydro-pyridin-5-carbonsäure-äthylester,
4-(3,4-Dichlor-2-thienyl)-6-hydroxymethyl-2-methyl-5-methylaminocarbonyl-1,4-dihydro-pyridin-3-carbonsäureäthylester,
6-Hydroxymethyl-2-methyl-5-methylaminocarbonyl-4-(2-nitro-phenyl)-1,4-dihydro-pyridin-3-carbonsäure-äthylester,
4-(2,3-Dimethyl-phenyl)-2-hydroxymethyl-6-methyl-3-methylaminocarbonyl-1,4-dihydro-pyridin-5-carbonsäure-äthylester,
3-Aethylamino-carbonyl-2-hydroxymethyl-6-methyl-4-(2-nitro-phenyl)-1,4-dihydro-pyridin-5-carbonsäure-äthylester,
3-Aethylaminocarbonyl-2-hydroxymethyl-6-methyl-4-(2-nitro-phenyl)-1,4-dihydro-pyridin-5-carbonsäure-methylester,

2-Hydroxymethyl-6-methyl-3-methylaminocarbonyl-4-(2-nitro-phenyl)-1,4-dihydro-pyridin-5-carbonsäure-methylester,

2-Hydroxymethyl-6-methyl-3-methylaminocarbonyl-4-(2-nitro-phenyl)-1,4-dihydro-pyridin-5-carbonsäure-isobutylester,

2-Hydroxymethyl-6-methyl-3-methylaminocarbonyl-4-(2-nitro-phenyl)-1,4-dihydro-pyridin-5-carbonsäure-(2-methoxy-äthyl)-ester,

2-Hydroxymethyl-6-methyl-3-methylaminocarbonyl-4-(2-difluormethoxy-phenyl)-1,4-dihydro-pyridin-5-carbonsäure-äthylester,

2-Hydroxymethyl-6-methyl-3-(2-dimethylaminoäthyl)-aminocarbonyl-4-(2-nitro-phenyl)-1,4-dihydro-pyridin-5-carbonsäure-äthylester.

2-Hydroxymethyl-6-methyl-3-methylaminocarbonyl-4-(2-furyl)-1,4-dihydro-pyridin-5-carbonsäure-äthylester,

2-Hydroxymethyl-6-methyl-3-methylaminocarbonyl-5-methylsulfonyl-4-(2-nitro-phenyl)-1,4-dihydro-pyridin,

2-Hydroxymethyl-6-methyl-3-methylaminocarbonyl-4-[2-(2-methoxyäthoxy)-phenyl]-1,4-dihydro-pyridin-5-carbonsäure-äthylester, oder der

2-Hydroxymethyl-6-methyl-3-methylaminocarbonyl-4-[2-(2-methylthioäthoxy)-phenyl-1,4-dihydro-pyridin-5-carbonsäure-äthylester.

Patentansprüche

1. 2-Hydroxymethyl-3-carbamoyl-1,4-dihydro-pyridin-Verbindungen der Formel

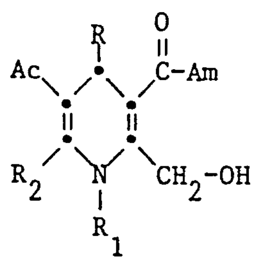

(I)

in welcher R einen carbocyclischen oder heterocyclischen Arylrest bedeutet, $R_1$ Wasserstoff oder unsubstituiertes oder substituiertes Niederalkyl bedeutet, $R_2$ Wasserstoff, Niederalkyl oder freies, veräthertes oder verestertes Hydroxy, funktionell abgewandeltes Carboxy oder freies oder substituiertes Amino enthaltendes Niederalkyl, funktionell abgewandeltes Carboxy oder freies oder substituiertes Amino bedeutet, wobei eine Aminogruppe $R_2$ mit einem Niederalkylrest $R_1$ verbunden sein kann, Ac den Acylrest einer Säure darstellt, und Am eine unsubstituierte oder monosubstituierte Aminogruppe bedeutet, mit der Massgabe, dass R von einem N-Oxido-pyridylrest verschieden ist, und mit der weiteren Massgabe, dass R von einem durch Halogen mit Atomnummer von höchstens 19 tetra- oder pentasubstituierten Phenylrest verschieden ist, wenn Ac für den Acylrest einer organischen Sulfonsäure steht, als Racematgemische, Racemate, optische Antipoden sowie Salze von Verbindungen der Formel I mit salzbildenden Eigenschaften.

2. Verbindungen der Formel I gemäss Anspruch 1, in welchen R für einen mono- oder bicyclischen, carbocyclischen Arylrest oder für einen fünf- oder sechsgliedrigen, ein bis und mit vierRingstickstoffatome, ein Ringsauerstoff- oder Ringschwefelatom, oder ein oder zwei Ringstickstoffatome zusammen mit einem Ringsauerstoff oder einem Ringschwefelatom als Ringglieder enthaltenden, monocyclischen Heteroarylrest, der gegebenenfalls einen ankondensierten Benzoring enthält, steht, und insbesondere Phenyl, Naphthyl, Pyrryl, Pyrazolyl, Imidazolyl, Triazolyl, Tetrazolyl, Furyl, Thienyl, Isoxazolyl, Oxazolyl, Oxadiazolyl, Isothiazolyl, Thiazolyl, Thiadiazolyl, Pyridyl, Pyridazinyl, Pyrimidinyl, Pyrazinyl, Triazinyl, Indolyl, Isoindolyl, Benzimidazolyl, Benzofuranyl, Benzothienyl, Chinolinyl oder Isochinolinyl bedeutet, wobei in diesen Resten Ringkohlenstoffatome gegebenenfalls durch Niederalkyl, Niederalkenyl, Niederalkinyl, Niederalkylen, Cycloalkyl, Phenyl und/oder Phenylniederalkyl, wobei Niederalkyl, Phenyl oder Phenylniederalkyl gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl und/oder Cyan, die cyclischen Reste auch Niederalkyl, das seinerseits wie angegeben substituiert sein kann, als Substituenten enthalten können, und/oder durch Hydroxy, Niederalkoxy, Halogenniederalkoxy, Niederalkenyloxy, Niederalkoxyniederalkoxy, Niederalkylthioniederalkoxy, Halogen, Niederalkinyloxy, Niederalkylendioxy, Niederalkanoyloxy, Nitro, Amino, N-Niederalkyl-amino, N,N-Diniederalkylamino, N-Niederalkyl-N-phenylniederalkyl-amino, Niederalkylenamino, Oxaniederalkylenamino, Thianiederalkylenamino und/oder Azaniederalkylenamino, worin das Azastickstoffatom durch Niederalkyl, Phenyl oder Phenylniederalkyl substituiert sein kann, wobei diese Substituenten Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl, und/oder Cyan als Substituenten enthalten können, und/oder durch Niederalkanoylamino, Azido, Carboxy, Niederalkoxycarbonyl, Carbamoyl, N-Niederalkyl-carbamoyl, N,N-Diniederalkyl-carbamoyl, Cyan, Sulfo, Aminosulfonyl, Niederalkylthio, Niederalkylsulfinyl und/oder Niederalkylsulfonyl, und/oder Ringstickstoffatome gegebenenfalls durch Niederalkyl, das als Substituenten gegebenenfalls Hydroxy, Niederalkoxy, Halogenniederalkoxy, Niederalkenyloxy, Niederalkinyloxy, Niederalkylendioxy, Niederalkanoyloxy, Halogen, Carboxy, Niederalkoxycarbonyl, Carbamoyl, N-Niederalkyl-carbamoyl, N,N-Diniederalkyl-carbamoyl oder Cyan als Substituenten enthalten kann, oder durch Hydroxy oder Oxido substituiert sein können, $R_1$ Wasserstoff, Niederalkyl, Diniederalkylamino-niederalkyl, Niederalkylenaminoniederalkyl, Morpholinoniederalkyl, Thiomorpholino-niederalkyl, Piperazinoniederalkyl oder 4-Niederalkyl-piperazino-niederalkyl darstellt, $R_2$ für Wasserstoff, Niederalkyl, Hydroxyniederalkyl, Niederalkoxyniederalkyl, Halogenniederalkyl, Niederalkoxycarbonylniederalkyl, Carbamoylniederalkyl, N-Niederalkylcarbamoylniederalkyl, N,N-Diniederalkylcarbamoylniederalkyl, Cyanniederalkyl, Aminoniederalkyl, Niederalkylaminoniederalkyl, Diniederalkylaminoniederalkyl, Niederalkylenaminoniederalkyl, Morpholinoniederalkyl, Thiomorpholinoniederalkyl, Piperazinoniederalkyl, 4-Niederalkyl-piperazino-niederalkyl, Niederalkoxycarbonyl, Carbamoyl, N-Niederalkylcarbamoyl, N,N-Diniederalkylcarbamoyl, Cyan, Amino, Niederalkylamino, Diniederalkylamino-niederalkylamino, Niederalkylenamino-niederalkylamino, Morpholino-niederalkylamino, Diniederalkylamino, Niederalkylenamino, (2-Oxo-1-imidazolidinyl)-niederalkylenamino, Morpholino, Thiomorpholino, Piperazino, 4-Niederalkyl-piperazino, 4-Niederalkanoyl-piperazino, 4-Benzoyl-piperazino, 4-Furoylpiperazino oder 4-Thienyl-piperazino steht, wobei eine Aminogruppe $R_2$ mit einem Niederalkylrest $R_1$ verbunden sein und mit diesem zusammen einen 1-Aza-niederalkylenrest bilden kann, dessen Stickstoffatom mit dem 6-Ringkohlenstoffatom des 1,4-Dihydro-pyridinrings verbunden ist, der Rest Ac für Niederalkanoyl, unsubstituiertes oder durch Niederalkyl, Niederalkoxy und/oder Halogen substituiertes Benzoyl, Niederalkylsulfonyl, unsubstituiertes oder durch Niederalkyl, Niederalkoxy und/oder Halogen substituiertes Phenylsulfonyl, Niederalkoxycarbonyl, Hydroxyniederalkoxycarbonyl, Niederalkoxyniederalkoxycarbonyl, Aminoniederalkoxycarbonyl, Niederalkylaminoniederalkoxycarbonyl, Diniederalkylaminoniederalkoxycarbonyl, N-Niederalkyl-N-phenylniederalkylamino-niederalkoxycarbonyl, Niederalkylenaminoniederalkoxycarbonyl, Morpholinoniederalkoxycarbonyl, Thiomorpholinoniederalkoxycarbonyl, Piperazinoniederalkoxycarbonyl, 4-Niederalkyl-piperazino-niederalkoxycarbonyl, unsubstituiertes oder durch Niederalkyl, Niederalkoxy und/oder Halogen substituiertes Phenylniederalkoxycarbonyl, Carbamoyl, N-Niederalkyl-carbamoyl, N,N-Diniederalkylcarbamoyl, N,N-Niederalkylencarbamoyl, Morpholinocarbonyl, Thiomorpholinocarbonyl, Piperazinocarbonyl oder 4-Niederalkyl-piperazinocarbonyl steht, und die Gruppe Am Amino, N-Niederalkyl-amino, N-(N'-Mono-niederalkylamino)-niederalkyl-amino, N-(N',N'-Diniederalkyl-amino)-niederalkyl-amino, (2-Niederalkylenamino-äthyl)-amino oder [2-(4-Niederalkyl-piperazino)-äthyl]-amino bedeutet, mit der Massgabe, dass R von einem N-Oxido-pyridylrest verschieden ist, und mit der weiteren Massgabe, dass R von einem durch Halogen mit Atomnummer von höchstens 19 tetra- oder pentasubstituierten Phenylrest verschieden ist, wenn Ac für den Acylrest einer organischen Sulfonsäure steht, oder Salze von solchen Verbindungen mit salzbildenden Gruppen.

3. Verbindungen der Formel I gemäss Anspruch 1, worin R für Phenyl, das gegebenenfalls durch Niederalkyl, Phenyl und/oder Phenylniederalkyl, wobei solche Reste ihrerseits Hydroxy, Niederalkoxy, Niederalkylendioxy, Halogen, Carboxy, Niederalkoxycarbonyl und/oder Cyan, die cyclischen Reste auch Niederalkyl als Substituenten enthalten können, und/oder durch Hydroxy, Niederalkoxy, Niederalkylendioxy, Niederalkoxyniederalkoxy, Niederalkylthioniederalkoxy, Halogen, Nitro, Amino, N-Niederalkylamino, N,N-Diniederalkylamino, Niederalkanoylamino, Carboxy, Niederalkoxycarbonyl, Carbamoyl, Cyan, Sulfo, Aminosulfonyl, Niederalkylthio, Niederalkylsulfinyl und/oder Niederalkylsulfonyl substituiert sein kann, oder für Pyrryl, Furyl, Thienyl oder Pyridyl, die gegebenenfalls wie ein Phenylrest R substituiert sein können, und insbesondere Niederalkyl, Niederalkoxy, Halogen und/oder gegebenenfalls durch Niederalkyl, Niederalkoxy, Halogen und/oder Nitro substituiertes Phenyl als Substituenten enthalten, $R_1$ Wasserstoff, Niederalkyl, Diniederalkylamino-niederalkyl, Niederalkylenaminoniederalkyl oder (4-Morpholino)-niederalkyl darstellt, wobei Diniederalkylamino, Niederalkylenamino bzw. 4-Morpholino durch mindestens zwei Kohlenstoffatome vom Ringstickstoffatom getrennt sind,

$R_2$ für Wasserstoff, Niederalkyl, Hydroxyniederalkyl, Halogenniederalkyl, Niederalkoxycarbonylniederalkyl, Cyanniederalkyl, Diniederalkylaminoniederalkyl, Niederalkoxycarbonyl, Cyan, Amino, (4-Morpholino)-niederalkylamino, Niederalkylenamino, (2-Oxo-1-imidazolidinyl)-niederalkylenamino oder 4-(2-Furoyl)-piperazino bedeutet, wobei eine Aminogruppe $R_2$ mit einem Niederalkylrest $R_1$ verbunden sein kann und mit diesem zusammen einen 1-Aza-niederalkylenrest bilden kann, dessen Azastickstoffatom mit dem 6-Ringkohlenstoffatom des 1,4-Dihydro-pyridinrings verbunden ist, der Rest Ac für Niederalkanoyl, Niederalkylsulfonyl, Niederalkoxycarbonyl, Hydroxyniederalkoxycarbonyl, Niederalkoxyniederalkoxycarbonyl, N,N-Diniederalkylaminoniederalkoxycarbonyl, N-Niederalkyl-N-phenylniederalkyl-amino-niederalkoxycarbonyl, N,N-Niederalkylenaminoniederalkoxycarbonyl, (4-Morpholino)-niederalkoxycarbonyl, Carbamoyl, N-Niederalkyl-carbamoyl, N,N-Diniederalkyl-carbamoyl, N,N-Niederalkylencarbamoyl, 4-Niederalkyl-piperazino-carbonyl oder 4-Morpholinocarbonyl steht, und der Rest Am für Amino, N-Niederalkyl-amino, N-(N'-Mono-niederalkylamino)-niederalkylamino, N-(N',N'-Diniederalkylamino)-niederalkyl-amino oder (2-Niederalkylenamino-äthyl)-amino steht, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kohlenstoffatome enthalten, mit der Massgabe, dass R von einem durch Halogen mit Atomnummer von höchstens 19 tetra- oder pentasubstituierten Phenylrest verschieden ist, wenn Ac für Niederalkylsulfonyl steht, oder Salze von solchen Verbindungen mit salzbildenden Gruppen.

4. Verbindungen der Formel I gemäss Anspruch 1, worin R für Phenyl oder Thienyl steht, das gegebenenfalls durch Niederalkyl, Hydroxy, Niederalkoxy, Niederalkylendioxy, Niederalkoxyniederalkoxy, Niederalkylthioniederalkoxy, Halogen, Trifluormethyl, Nitro, Niederalkanoylamino und/oder Cyan mono-, di- oder trisubstituiert sein kann, $R_1$ Wasserstoff, Niederalkyl, 2-(Diniederalkylamino)-niederalkyl, 2-(Niederalkylenamino)-niederalkyl oder 2-(4-Morpholino)-niederalkyl bedeutet, $R_2$ Niederalkyl, Hydroxyniederalkyl, Halogenniederalkyl, 2-(Diniederalkylamino)-niederalkyl, Niederalkoxycarbonyl,

Cyan, Amino, (4-Morpholino)-niederalkylamino, Niederalkylenamino, (2-Oxo-1-imidazolidinyl)-niederalkylenamino oder 4-(2-Furoyl)-piperazino bedeutet, wobei eine Aminogruppe $R_2$ mit einem Niederalkylrest $R_1$ verbunden und zusammen mit diesem einen 1-Azaniederalkylenrest bilden kann, dessen Azastickstoffatom mit dem 6-Ringkohlenstoffatom des 1,4-Dihydropyridinrungs verbunden ist, der Rest Ac Niederalkanoyl, Niederalkylsulfonyl, Niederalkoxycarbonyl, 2-Niederalkoxy-niederalkoxycarbonyl, Diniederalkylamino-niederalkoxycarbonyl, N-Niederalkyl-N-phenylniederalkyl-amino-niederalkoxycarbonyl, Carbamoyl, N-Niederalkylcarbamoyl, N,N-Diniederalkyl-carbamoyl, N,N-Niederalkylenamino-carbonyl, 4-Niederalkyl-piperazino-carbonyl oder 4-Morpholino-carbonyl bedeutet, und der Rest Am für Amino, N-Niederalkylamino, N-(N'-Mono-niederalkylamino)-niederalkyl-amino, z.B. 2-(Methylamino)-äthylamino, oder N-(N',N'-Diniederalkyl-amino)-niederalkyl-amino, z.B. 2-(Dimethylamino)-äthylamino steht, wobei Niederalkyl, Niederalkoxy und Niederalkanoyl bis und mit 4 Kohlenstoffatome, Niederalkylen 4 oder 5 Kettenkohlenstoffatome, 1-Aza-niederalkylen 2 oder 3 Kettenkohlenstoffatome enthalten, oder Salze von solchen Verbindungen mit salzbildenden Gruppen.

5. Verbindungen der Formel I gemäss Anspruch 1, worin R für unsubstituiertes oder vorzugsweise durch Niederalkyl, z.B. Methyl, Halogenniederalkoxy, z.B. Difluormethoxy, Niederalkoxy, z.B. Methoxy, Niederalkoxyniederalkoxy, z.B. 2-Methoxyäthoxy, Niederalkylthioniederalkoxy, z.B. 2-Methylthioäthoxy, Halogen, z.B. Fluor oder Chlor, Trifluormethyl, Nitro und/oder Cyan mono- oder disubstituiertes Phenyl oder 2- oder 3-Thienyl steht, $R_1$ Wasserstoff oder 2-(4-Morpholino)-äthyl bedeutet, $R_2$ Methyl, Hydroxymethyl, Niederalkoxycarbonyl, z.B. Methoxycarbonyl, Cyan oder Amino bedeutet, der Rest Ac Niederalkanoyl, z.B. Acetyl, Niederalkylsulfonyl, z.B. Aethylsulfonyl, Niederalkoxycarbonyl, z.B. Methoxycarbonyl oder Aethoxycarbonyl, Carbamoyl, N-Niederalkylcarbamoyl, z.B. N-Methylcarbamoyl oder N,N-Diniederalkyl-carbamoyl, z.B. N,N-Dimethylcarbamoyl darstellt und die Gruppe Am für Amino oder N-Niederalkyl-amino, z.B. Methylamino

steht, wobei Niederalkyl, Niederalkoxy und Niederalkanoyl bis und mit 4 Kohlenstoffatome enthalten, oder Salze von solchen Verbindungen mit salzbildenden Gruppen.

6. 2-Hydroxymethyl-6-methyl-3-methylaminocarbonyl-4-(2-nitro-phenyl)-1,4-dihydro-pyridin-5-carbonsäure-äthylester.

7. 2-Hydroxymethyl-6-methyl-3-methylaminocarbonyl-4-(3-nitro-phenyl)-1,4-dihydro-pyridin-5-carbonsäure-äthylester.

8. 2-Hydroxymethyl-6-methyl-3-methylaminocarbonyl-4-(4-methylamino-2,3,5,6-tetrafluor-phenyl)-1,4-dihydro-pyridin-5-carbonsäure-äthylester.

9. 4-(3-Cyan-phenyl)-2-hydroxymethyl-6-methyl-3-methylaminocarbonyl-1,4-dihydro-pyridin-5-carbonsäure-äthylester.

10. 4-(2,3-Dichlor-phenyl)-2-hydroxymethyl-6-methyl-3-methylamino-carbonyl-1,4-dihydro-pyridin-5-carbonsäure-äthylester.

11. 2-Hydroxymethyl-6-methyl-3-methylaminocarbonyl-4-(2-trifluormethyl-phenyl)-1,4-dihydro-pyridin-5-carbonsäure-äthylester.

12. 4-(3,4-Dichlor-2-thienyl)-6-hydroxymethyl-2-methyl-5-methylamino-carbonyl-1,4-dihydro-pyridin-3-carbonsäure-äthylester.

13. 4-(2,3-Dimethyl-phenyl)-2-hydroxymethyl-6-methyl--3-methylamino-carbonyl-1,4-dihydro-pyridin-5-carbonsäure-äthylester.

14. 3-Aethylaminocarbonyl-2-hydroxymethyl-6-methyl-4-(2-nitro-phenyl)-1,4-dihydro-pyridin-5-carbonsäure-äthylester.

15. 3-Aethylaminocarbonyl-2-hydroxymethyl-6-methyl-4-(2-nitro-phenyl)-1,4-dihydro-pyridin-5-carbonsäure-methylester.

16. 2-Hydroxymethyl-6-methyl-3-methylaminocarbonyl-4-(2-nitro-phenyl)-1,4-dihydro-pyridin-5-carbonsäure-methylester.

17. 2-Hydroxymethyl-6-methyl-3-methylaminocarbonyl-4-(2-furyl)-1,4-dihydro-pyridin-5-carbonsäure-äthylester.

18. 2-Hydroxymethyl-6-methyl-3-methylaminocarbonyl-5-methylsulfonyl-4-(2-nitro-phenyl)-1,4-dihydro-pyridin.

19. 2-Hydroxymethyl-6-methyl-3-methylaminocarbonyl-4-[2-(2-methoxy-äthoxy)-phenyl]-1,4-dihydro-pyridin-5-carbonsäure-äthylester.

20. 2-Hydroxymethyl-6-methyl-3-methylaminocarbonyl-4-[2-(2-methylthioäthoxy)-phenyl]-1,4-dihydropyridin-5-carbonsäure-äthylester.

21. Die Verbindungen der Ansprüche 1-20 als pharmakologisch aktive, insbesondere blutdrucksenkende und coronardilatierende Verbindungen.

22. Die Verbindungen der Ansprüche 1-20 zur Behandlung des menschlichen oder tierischen Körpers.

23. Verwendung der Verbindungen der Ansprüche 1-20 zur Behandlung von cardiovaskulären Krankheitszuständen.

24. Pharmazeutische Präparate enthaltend eine Verbindung der Ansprüche 1-20.

25. Verwendung der Verbindungen der Ansprüche 1-20 zur Herstellung von pharmazeutischen Präparaten.

26. Verfahren zur Herstellung von pharmazeutischen Präparaten, dadurch gekennzeichnet, dass man Verbindungen der Ansprüche 1- 20 gegebenenfalls unter Beimischung von Hilfsstoffen, zu pharmazeutischen Präparaten verarbeitet.

27. Verfahren zur Herstellung von Verbindungen der Formel I gemäss Anspruch 1, als Racematgemische, Racemate, optische Antipoden oder Salzen von solchen Verbindungen mit salzbildenden Gruppen, dadurch gekennzeichnet, dass man

a) eine Verbindung der Formel

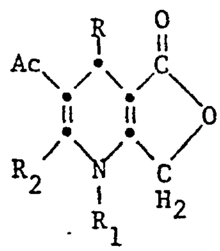

(II)

mit einer Verbindung der Formel H-Am (III) umsetzt, oder

b) in einer Verbindung der Formel

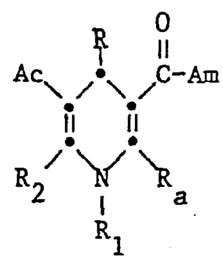

(IV)

in welcher $R_a$ einen in die Carbinolgruppe der Formel $-CH_2-OH$ überführbaren Rest bedeutet, den Rest $R_a$ in letztere überführt, oder

c) in einer Verbindung der Formel

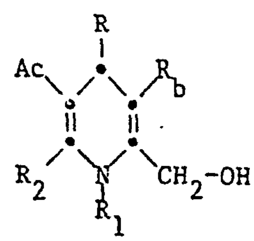

(V)

worin $R_b$ einen in die Carbamoylgruppe der Formel -C(=O)-Am überführbaren Rest darstellt, den Rest $R_b$ in letztere überführt, oder

d) in einer Verbindung der Formel

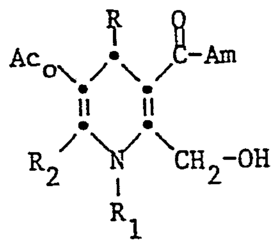

(VI)

worin $Ac_o$ einen in die Gruppe Ac überführbaren Rest darstellt, diesen in letztere überführt, oder

e) eine Verbindung der Formel

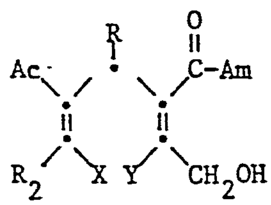

(VII),

worin einer der Reste X und Y für die Gruppe der Formel $-NH-R_1$ steht und der andere Hydroxy oder die Gruppe der Formel $-NH-R_1$ bedeutet, oder ein Tautomeres davon oder ein entsprechendes Tautomerengemisch, ringschliesst,
wobei in den obigen Ausgangsstoffen der Formeln II bis VII, die, sofern sie salzbildende Eigenschaften aufweisen, auch in Form ihrer Salze verwendet werden können, die Reste R, $R_1$, $R_2$, Ac und Am die unter der Formel I gegebenen Bedeutungen haben, und, wenn erwünscht, eine erhaltene Verbindung der Formel I in eine andere Verbindung der Formel I umwandelt, und/oder, wenn erwünscht, ein erhaltenes Racematgemisch in die Racemate, oder ein erhaltenes Racemat in die optischen Antipoden auftrennt, und/oder, wenn erwünscht, ein erhaltenes Salz in die freie Verbindung oder in ein anderes Salz umwandelt, und/oder, wenn erwünscht, eine erhaltene freie Verbindung der Formel I mit salzbildenden Eigenschaften in ein Salz umwandelt, und/oder, wenn erwünscht, ein erhaltenes Gemisch von Isomeren in die einzelnen Isomeren auftrennt.

28. Die nach dem Verfahren des Anspruchs 27 erhältlichen Verbindungen.

FO 7.4 EJA/gs*/cw*

0111453

Europäisches Patentamt

**EUROPÄISCHER RECHERCHENBERICHT**

Nummer der Anmeldung

EP 83 81 0572

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | FR-A-2 435 471 (FUJISAWA)<br>* Ansprüche; Beispiele 4,11 *<br>--- | 1-6,21 -28 | C 07 D 211/90<br>C 07 D 491/04<br>C 07 D 409/04<br>C 07 D 405/04<br>C 07 D 401/04<br>A 61 K 31/455//<br>(C 07 D 491/04<br>C 07 D 307/00<br>C 07 D 221/00 ) |
| A | FR-A-2 187 349 (BAYER)<br>* Ansprüche; Beispiele *<br>--- | 1-6,21 -28 | |
| X,P | EP-A-0 083 315 (CIBA-GEIGY)<br>* Ansprüche; Beispiel 10 *<br>--- | 1-6,21 -28 | |
| P,Y | EP-A-0 071 819 (BAYER)<br>* Ansprüche *<br>--- | 1-6,21 -28 | **RECHERCHIERTE SACHGEBIETE (Int. Cl. 3)** |
| P,Y | EP-A-0 069 033 (RHONE POULENC)<br>* Ansprüche; Beispiel 5, Verbindung 88 *<br>----- | 1-6,21 -28 | C 07 D 211/00<br>C 07 D 491/00<br>C 07 D 401/00<br>C 07 D 405/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 18-02-1984 | NUYTS A.M.K.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82